# EUROPÄISCHE PATENTANMELDUNG

(11) **EP 2 031 067 A1**
(43) Veröffentlichungstag der Anmeldung: **04.03.2009**
(21) Anmeldenummer: 07017025.3
(22) Anmeldetag: 30.08.2007
(51) Int. Cl.: C12N 15/76

(54) **Herstellung von genetisch modifizierten Aktinomyceten durch Rekombination**

(71) Anmelder: Albert-Ludwigs-Universität Freiburg, 79098 Freiburg (DE)
(72) Erfinder: Bechthold, Andreas, Prof. Dr., 79227 Schallstadt (DE); Luzhetsjyy, Andriy, Dr., 79104 Freiburg (DE); Petzke, Lutz, 79100 Freiburg (DE); Welle, Elisabeth, 79395 Neuenburg (DE); Fedoryshyn, Marta, 79104 Freiburg (DE); Daum, Martina, 79114 Freiburg (DE); Herrmann, Simone, 79100 Freiburg (DE)
(74) Vertreter: Kalhammer, Georg

(57) **Zusammenfassung**

Die vorliegende Erfindung betrifft die rekombinante Modifizierung von Mikroorganismen, insbesondere Aktinomyceten. Diese erfolgt unter Verwendung der Rekombinationssysteme Dre-rox, Cre-Iox und Flp-Frt. Die vorliegende Erfindung stellt neue Nukleotidsequenzen für die Rekombinasen der vorstehend genannten Rekombinationssysteme zur Verfügung. Hierbei wurden synthetische Gene hergestellt, die die von Aktinomyceten bevorzugten Codons aufweisen und daher in diesen Wirtsorganismen verwendet werden können.

Mit dem erfindungsgemäßen Verfahren zur Modifizierung von Mikroorganismen ist es möglich Überproduktionsstämme zu erzeugen, die von wirtschaftlicher Bedeutung für die Herstellung von medizinischen Wirkstoffen sind.

## Beschreibung

Die vorliegende Erfindung betrifft in Wirtszellen replizierbare Nukleinsäuremoleküle, die neue DNA-Sequenzen enthalten, die für die Dre-, Cre- oder Flp-Rekombinase kodieren. Des Weiteren betrifft die Erfindung ein Rekombinationssystem zum Verändern von DNA-Fragmenten (Ausschneiden, Integrieren, Invertieren oder Translokieren) des Genoms (Ziel-DNA) von Aktinomyceten. Zudem betrifft die Erfindung ein Verfahren zur Herstellung von modifizierten Aktinomyceten, sowie die modifizierten Mikroorganismen selbst, die durch dieses Verfahren hergestellt wurden.

Fast die Hälfte aller Arzneistoffe auf dem Markt sind Naturstoffe oder sind von solchen abgeleitet. Eine Vielzahl der Wirkstoffe wird unter Verwendung von Mikroorganismen hergestellt. Einige erfolgreiche Beispiele auf dem Markt sind die Antibiotika Erythromycin, Tetracyclin oder die Immunsuppressiva Cyclosporin, FK506 und Rapamycin.

Ein Verfahren zur Herstellung von Naturstoffen bzw. Sekundärmetaboliten ist die "kombinatorische Biosynthese". In diesem Verfahren werden künstliche Biosynthesewege aus einer großen, ständig wachsenden Sammlung an Genen geschaffen, die an Naturstoffsynthesen beteiligt sind. Dazu ist zunächst die oftmals arbeitsintensive und zeitaufwendige Konstruktion von Mutanten eines Bakterienstammes notwendig. Diese Mikroorganismen sollen eine Überproduktion an Sekundärmetaboliten aufweisen und damit eine kosteneffiziente Methode zur Herstellung von Wirkstoffen ermöglichen.

Die genetische Modifizierung von Mikroorganismen kann bekannterweise durch Rekombination erfolgen. Die Rekombinasen der Cre-lox- (bzw. Cre-loxP), Flp-Frt- und Drerox-Systeme können das Ausschneiden oder Integrieren sowie die Inversion oder Translokation von DNA-Fragmenten katalysieren, welche sich zwischen spezifischen Erkennungssequenzen befinden. Die Rekombinasen Cre, Flp und Dre benötigen hierbei keine zusätzlichen Cofaktoren (Buchholz et al., Nucleic Acids Res. 1996, 24, 3118-3119; Huang et al., J. Bacteriol. 1997, 179, 6076-6083; Shimshek et al., Genesis, 2002, 32, 19-26).

Bei einem derartigen Rekombinationsverfahren ist es notwendig, die Nukleinsäuren, die für die Rekombinasen kodieren, in die Wirtszelle einzubringen und zu exprimieren. Dies kann beispielsweise mittels eines Plasmids erfolgen, wobei bei Verwendung von induzierbaren Promotoren die Expression der Rekombinasen gesteuert werden kann. Die Rekombinasen können dann die Veränderung des Genoms der Wirtszelle bewirken. Die Art der Rekombination wird hierbei durch die Anordnung der Erkennungssequenzen loxP, Frt und rox zueinander auf einem DNA-Abschnitt bestimmt.

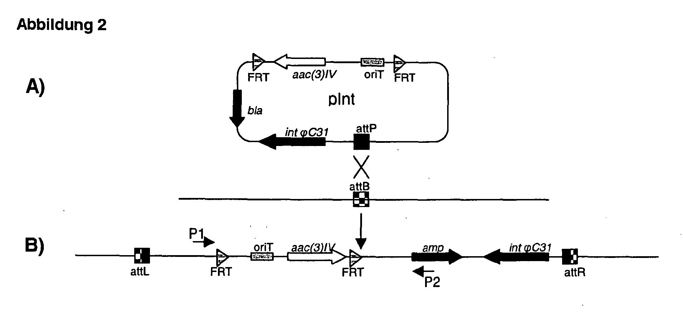

Das Einbringen von Erkennungssequenzen in das Genom der Wirtszelle kann durch übliche Verfahren, beispielsweise durch die homologe Rekombination erfolgen. Bei Anwesenheit von zwei gleichen Erkennungssequenzen kann die Rekombinase das Ausschneiden des DNA-Fragments, das sich zwischen diesen Erkennungssequenzen befindet, bewirken, wobei das ausgeschnittene DNA-Fragment anschließend cyclisieren kann. Des Weiteren ist es möglich, cyclische Nukleinsäuremoleküle, die eine Erkennungssequenz aufweisen, z.B. in Form eines Plasmids, in das Genom der Wirtszelle einzufügen. Hierzu ist im Genom der Wirtszelle ebenfalls eine Erkennungssequenz notwendig, woraufhin die Rekombinase einen Austausch der Nukleinsäurestränge an den Erkennungssequenzen bewirkt. Auf ähnliche Weise erfolgt das Einfügen eines cyclischen Nukleinsäuremoleküls in *E. coli*-Stämmen unter Verwendung der Erkennungssequenzen attP (Erkennungssequenz eines Bakteriophagen) und attB (bakterielle Erkennungssequenz, siehe Abbildung 2). Dieser Vorgang wird durch zwei Enzyme katalysiert: dem Phagenprotein Integrase und dem bakteriellen *Integration Host Factor* (IHF). Das Phagengenom liegt nach der Integration als Prophage im bakteriellen Genom vor, und wird von den neugebildeten Rekombinationsstellen flankiert.

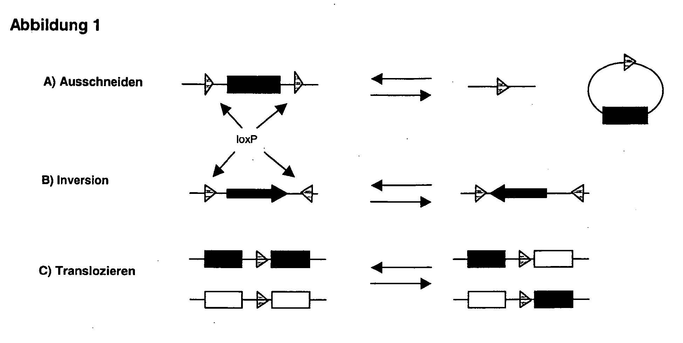

Abbildung 1 zeigt das Ausschneiden eines Genfragments (A), das Invertieren eines Genfragments (B) sowie die Translokation eines Genfragments (C). Hierbei werden richtungsabhängige IoxP-Erkennungssequenzen und die Cre-Rekombinase verwendet. Die IoxP-Erkennungssequenzen bestehen aus einer Basenregion (acht Basenpaare), die von zwei invertierten Wiederholungen (inverted repeats, 13 Basenpaare) flankiert wird, die als Erkennungssequenzen für die DNA-Bindung von Cre dienen (Mack et al., Nucleic Acid Res., 1992, 20, 4451-4455; Hoess et al., J. Mol. Biol., 1990, 216, 873-882). Das Rekombinationsereignis ist lediglich von diesen beiden Bestandteilen abhängig und wird mit absoluter Zuverlässigkeit durchgeführt. Das Flp-Frt-System basiert auf den gleichen Mechanismen.

Von besonderer Bedeutung für die Rekombination mittels Rekombinasen ist die Fähigkeit der Wirtszelle, die Rekombinasen zu exprimieren. Die Dre-, Flp- und Cre-vermittelte Rekombination ist sowohl in *E. coli* als auch in eukaryotischen Zellen einschließlich *Drosophila-,* Maus-, Mais-, Reis-, *Arabidopsis-* und Tabakzellen möglich (Campo et al., Appl. Environ. Microbiol., 2002, 68, 2359-2367; Golic et al., Cell, 1989, 59, 499-509; Posfai et al., Nucleic Acids Res., 1994, 22, 2392-2398; Sauer and McDermott, Nucleic Acid Res. 2004, 18, 6086-6095).

Die oben dargestellten Rekombinationssysteme können mittels Plasmiden in Aktinomyceten eingebracht und dort vervielfältigt werden. Hierbei können die Nukleotidsequenzen, die für die Flp-, Cre-, und Dre-Rekombinasen kodieren, zusammen mit den notwendigen promotoren und Replikationsursprüngen in ein geeignetes Plasmid kloniert werden. Dieses Plasmid kann beispielsweise mittels Konjugation in einen Mikroorganismus eingebracht und dort vervielfältigt werden. Die Rekombinasen werden dann in den Wirtsorganismen exprimiert.

Die in den Wirtsorganismus eingebrachten Rekombinationssysteme sind dann in der Lage, beispielsweise DNA-Fragmente in das Genom eines Wirtsorganismus einzufügen oder aus diesem herauszuschneiden. Auf diese Weise ist es beispielsweise möglich, Gene des Wirtsorganismus an- oder auszuschalten, wobei dies die Produktion der gewünschten Wirkstoffe bzw. Sekundärmetaboliten beeinflusst.

Besonders geeignete Mikroorganismen für die Produktion von Naturstoffen sind die Aktinomyceten und dabei besonders Stämme, die zur Gattung Streptomyces gehören. Kürzlich wurde ein Verfahren zur Verwendung des Cre-Iox-Systems in *Streptomyces coelicolor* entwickelt (Khodakaramian et al., Nucleic Acids Res., 2006, 34, e20. Dieses System ist jedoch nur bedingt in anderen Stämmen einsetzbar. Es basiert auf dem Phagen ϕC31, der nicht alle Stämme transfizieren kann. Außerdem ist der Umgang mit Phagen im Zusammenhang mit Streptomyceten nicht einfach, so dass eine Routineanwendung nicht möglich ist. Versuche, die Flp-Rekombinasen in Aktinomyceten zu exprimieren, waren bisher nicht erfolgreich (Khodakaramian et al., Nucleic Acids Res., 2006, 34, e20).

Des Weiteren ist die heterologe Expression von Proteinen oft schwierig. Beobachtet wird, dass Transkription und Translation erfolgreich verlaufen, die Faltung der Proteine jedoch nicht gelingt. Proteasen können die fremden Proteine erkennen und zerstören. Überraschenderweise war es nun möglich, die oben dargestellten Rekombinationssysteme an Aktinomyceten dadurch anzupassen, dass die Codon-Usage der Gene, die für die Rekombinasen kodieren, optimiert wurde. Durch die Verwendung von Codon-Usage optimierten synthetischen Genen ist es nun möglich, eine Expression der Gene in Aktinomyceten zu ermöglichen. Dadurch werden die vorteilhaften Rekombinationsverfahren für die Aktinomyceten anwendbar.

Des Weiteren ist es nicht nur von Nachteil, wenn ein System zur Rekombination nur in einem speziellen Stamm anwendbar ist, sondern es ist auch von Nachteil wenn nur ein System zur Rekombination in diesem Stamm funktioniert. Da nicht beliebig viele Erkennungssequenzen in ein Chromosom eingebaut werden können - dies führt zu unerwünschten sekundären Effekten -, sind komplexe Modifikationen, die den Austausch mehrerer Genfragmente benötigen, nicht mit einem Rekombinationssystem alleine möglich. Es wäre daher wünschenswert, komplexe Modifikationen an Mikroorganismen dadurch zu ermöglichen, dass verschiedene Rekombinationsverfahren in Kombination eingesetzt werden können.

Eine Aufgabe der vorliegenden Erfindung ist es daher, die Cre-Iox-, Flp-Frt-, Dre-rox-Rekombinationssysteme für die Gattung der Aktinomyceten anwendbar zu machen. Insbesondere soll hierbei eine Kombination von verschiedenen Rekombinationssystemen zur Herstellung eines Überproduktionsstammes möglich werden.

Diese Aufgabe wird daher durch die erfindungsgemäßen neuen Nukleinsäuresequenzen, die für die Rekombinasen kodieren, gelöst. Die vorliegende Erfindung betrifft deshalb in Wirtszellen replizierbare Nukleinsäuremoleküle.

In einer bevorzugten Ausführungsform ist das in Wirtszellen replizierbare Nukleinsäuremolekül dadurch gekennzeichnet, dass es mindestens eine Nukleinsäuresequenz, ausgewählt aus der Gruppe bestehend aus:
i) DNA-Sequenzen, die über die ganze Länge mindestens 80% Identität zu der Nukleotidsequenz SEQ ID Nr. 1 aufweisen und für eine Dre-Rekombinase kodieren,
ii) DNA-Sequenzen, die über die ganze Länge mindestens 80% Identität zu der Nukleotidsequenz SEQ ID Nr. 2 aufweisen und für eine Cre-Rekombinase kodieren,
iii) DNA-Sequenzen, die über die ganze Länge mindestens 80% Identität zu der Nukleotidsequenz SEQ ID Nr. 3 aufweisen und für eine FLP-Rekombinase kodieren,
enthält.

In einer weiteren Ausführungsform ist das in Wirtszellen replizierbares Nukleinsäuremolekül dadurch gekennzeichnet, dass die Nukleotidtrippletts aus der Gruppe i) - iii) zu mindestens 70% solche Codons darstellen, die von Aktinomyceten bevorzugt sind.

Das in Wirtszellen replizierbare Nukleinsäuremolekül kann dadurch bevorzugt in Wirtszellen, wie einem *Escherichia coli-* und einem oder mehr *Aktinomyceten*-Stämmen repliziert werden. Bevorzugt kann es in einem Aktinomyceten-Stamm vervielfältigt und exprimiert werden.

In einer weiter bevorzugten Ausführungsform ist das in Wirtszellen replizierbare Nukleinsäuremolekül dadurch gekennzeichnet, dass die Wirtszellen einen *Streptomyceten -* Stamm darstellen, wobei *Streptomyces lividans,* Streptomyces coelicolor, Streptomyces albus, *Streptomyces cinnamonensis, Actinoplanes* sp. SE50/110, *Streptomyces galilaeus, Streptomyces nodosus, Actinosynnema pretiosum subsp. Auranticum, Streptomyces virido-chromogenes* Tü57, *Streptomyces echinatus, Streptomyces avermitilis* MA-4680, *Micromonospora echinospora, Streptomyces sp. FR-008, Streptomyces chartreusis, Streptomyces bikiniensis, Streptomyces roseochromogenes subsp. oscitans* DS12.976, *Streptomyces griseus* subsp. griseus, *Streptomyces neyagawaensis, Streptomyces olindensis, Streptomyces rishiriensis* DSM 40489, *Streptomyces venezuelae, Streptomyces peucetius, Streptomyces olivaceus* Tü2353, *Saccharopolyspora erythraea, Micromonospora carbonacea* var. Africana ATCC39149, *Streptomyces griseoflavus* Gö3592, *Streptomyces ambofaciens, Streptomyces violaceoruber* Tü22, *Amycolatopsis orientalis, Amycolatopsis orientalis, Amycolatopsis mediterranei (balhimycina)* DSM5908, *Micromonospora echinospora* ATCC15835, *Streptomyces griseoruber, Streptomyces aureofaciaens* Tü11, *Streptomyces antibioticus* Tü99, *Streptomyces antibioticus* Tü1718, *Streptomyces sp.* Tü2187, *Streptomyces sp.* Tü1156, *Streptomyces sp.* FU107, *Streptomyces sp.* FU36, *Streptomyces venezuelae* ISP5230, *Streptomyces kanamyceticus, Streptomyces cyanogenus* S136, *Streptomyces aureofaciens* Tü117, *Streptomyces rochei, Streptomyces globisporus* 1912, *Streptomyces* sp. AM-7161, *Micromonospora megalomicea, Streptomyces argillaceus, Streptomyces carzinostaticus* ATCC 15944, *Streptomyces fradiae* NCIB 8233, *Streptomyces spheroides,* NCIMB 11891, *Streptomyces noursei* ATCC11455, *Streptomyces antibioticus, Actinoplanes teichomyceticus, Streptomyces natalensis, Streptomyces* sp. Tü 6071, *Streptomyces diastato-chromogenes* Tü6028, *Lechevalieria aerocolonigenes* ATCC39243, *Saccharothrix espanaensis, Micromonospora* sp. Tü 6368, *Streptomyces antibioticus* Tü6040, *Streptomyces nogalater, Sorangium cellulosum* So ce12, *Saccharopolyspora, Spinosa, Streptomyces* sp. TP-A0274, *Streptomyces steffisburgensis* NRRL 3193, *Streptomyces griseus, Streptomyces fradice, Streptomyces fradiae* Tü2717, und *Streptomyces halstedii* HC-34 bevorzugt sind.

In einer weiteren Ausführungsform ist das in Wirtszellen replizierbare Nukleinsäuremolekül dadurch gekennzeichnet, dass es ein Plasmid darstellt.

In einer anderen Ausführungsform ist das in Wirtszellen replizierbare Nukleinsäuremolekül dadurch gekennzeichnet, das Plasmid einen ErmE- oder tipA-Promotor aufweist.

Weiter bevorzugt ist das in Wirtszellen replizierbare Nukleinsäuremolekül dadurch gekennzeichnet, dass das Plasmid ausgewählt ist aus der Gruppe bestehend aus: pUWL-T-FLP, pUWL-A-FLP, pUWL-H-FLP, pNL1-FLP, pML1-FLP und pAL1-FLP, pUWL-T-Dre, pUWL-A-Dre, pUWL-H-Dre, pNL1-Der, pML1-Dre und pAL1-Dre, pUWL-T-Cre, pUWL-A-Cre, pUWL-H-Cre, pNL1-Cre, pML1-Cre und pAL1-Cre.

Des Weiteren betrifft die Erfindung ein Rekombinationssystem zum Ausschneiden, Integrieren, Invertieren oder Translokieren von DNA-Fragmenten aus/in dem/das Genom (Ziel-DNA) von Acetomyceten, dadurch gekennzeichnet, dass es mindestens ein erfindungsgemäßes Nukleinsäuremolekül und mindestens eine Erkennungssequenz in der Ziel-DNA aufweist. Weiter bevorzugt umfasst das Rekombinationssystem ein DNA-Fragment, das in das Genom der Aktinomyceten eingefügt werden soll. Weiter bevorzugt wird das einzufügende DNA-Fragment mittels eines Plasmids in die Wirtszelle eingebracht. Bevorzugterweise umfasst das Plasmid hierbei eine Erkennungssequenz, die einer Erkennungssequenz in der Ziel-DNA entspricht. Weiter bevorzugt umfasst das Plasmid zusätzlich die notwendigen Replikationsursprünge für Aktinomyceten und für *E. coli,* weiter bevorzugt für Aktinomyceten. Noch weiter bevorzugt weist das einzufügende DNA-Fragment eine Länge von 1000 Basenpaaren auf.

In einer anderen Ausführungsform ist das Rekombinationssystem dadurch gekennzeichnet, dass die Erkennungssequenz ausgewählt ist aus der Gruppe bestehend aus: FRT-, IoxP-, und rox-Erkennungssequenzen.

In einer weiter bevorzugten Ausführungsform ist das Rekombinationssystem dadurch gekennzeichnet, dass die Ziel-DNA mindestens zwei Erkennungssequenzen, ausgewählt aus der Gruppe bestehend aus: FRT-, IoxP-, und rox-Erkennungssequenzen, enthält. Weiter bevorzugt enthält die Ziel-DNA zwei unterschiedliche Erkennungssequenzen aus der vorstehend genannten Gruppe.

In einer weiter bevorzugten Ausführungsform ist das Rekombinationssystem dadurch gekennzeichnet, dass die Nukleinsäuremoleküle, die für die Rekombinasen kodieren, in einem Plasmid enthalten sind und das Plasmid in einen *Escherichia coli*-Stamm vermehrt und/oder *Aktinomyceten*-Stamm eingeführt und dort repliziert und/oder exprimiert wird.

Des Weiteren betrifft die Erfindung ein Verfahren zur Herstellung von modifizierten Aktinomyceten, umfassend die folgenden Schritte:
a) Einfügen von mindestens einer Erkennungssequenz in das Genom (Ziel-DNA) des Aktinomyceten, wobei die Erkennungssequenzen ausgewählt sind aus der Gruppe bestehend aus Frt-, IoxP- und rox-Erkennungssequenzen,
b) Einbringen mindestens eines Plasmids in den Aktinomyceten, wobei das Plasmid ein replizierbares Nukleinsäuremolekül gemäß einem der Ansprüche 1-8 darstellt, und
c) Vervielfältigen des Plasmids aus b) und Exprimieren der Rekombinase, die für die Erkennungssequenz(en) der Ziel-DNA spezifisch ist, ein DNA-Fragement der Ziel-DNA ausschneidet, translokiert, invertiert oder ein DNA-Fragment in die Ziel-DNA einfügt.

In einer anderen Ausführungsform ist das Verfahren dadurch gekennzeichnet, dass mindestens zwei Erkennungssequenzen, ausgewählt aus der Gruppe bestehend aus: Frt-, IoxP-, und rox-Erkennungssequenzen in die Ziel-DNA eingefügt werden.

Weiter bevorzugt ist das Verfahren dadurch gekennzeichnet, dass mindestens zwei unterschiedliche erfindungsgemäße Nukleinsäuremoleküle verwendet werden. Hierbei ist es bevorzugt, dass mindestens zwei verschiedene DNA-Fragmente durch mindestens zwei verschiedene erfindungsgemäße Rekombinationssysteme in die Ziel-DNA eingefügt oder aus dieser herausgeschnitten werden können.

Eine weiter bevorzugte Ausführungsform ist die Verwendung eines Rekombinationssystems zum Ausschneiden, Integrieren, Invertieren oder Translokieren von DNA-Fragmenten in Aktinomyceten.

Des Weiteren betrifft die Erfindung einen modifizierten Mikroorganismus, hergestellt durch das erfindungsgemäße Verfahren.

Die erfindungsgemäßen Nukleinsäuremoleküle ermöglichen erstmals das Herstellen von Plasmiden, die in Aktinomyceten und Streptomyceten vervielfältigt werden können. Die Expression der neuen erfindungsgemäßen Nukleotidsequenzen bzw. Nukleinsäuremoleküle in den Wirtsorganismen führt zu den Rekombinasen, die eine Veränderung des Genoms der Wirtszelle durch Rekombination ermöglichen.

Besonders vorteilhaft ist dabei, dass die Rekombinationssysteme Flp-Frt, Cre-Iox und Drerox nun für alle Aktinomyceten geeignet sind. Dadurch wird es insbesondere möglich, die verschiedenen Systeme zur Rekombination miteinander zu kombinieren um die Möglichkeiten hinsichtlich des Austausches mehrerer Fragmente in einem Genom zu erweitern.

Das erfindungsgemäße Verfahren ermöglicht des Weiteren auf vorteilhafte Weise die Deletion und das gleichzeitige Klonieren von Biosynthesegenclustern. Dies wiederum ermöglich die schnelle Generierung von Mutanten und somit die schnelle und effiziente Generierung neuer Naturstoffe.

Das erfindungsgemäße Verfahren ermöglicht, dass Überproduktionsstämme, die Naturstoffe, Wirkstoffe bzw. Sekundärmetaboliten produzieren, in kürzester Zeit hergestellt werden können.

Im Nachfolgenden werden die Rekombinationssysteme mit den erfindungsgemäßen Nukleotidsequenzen bzw. Nukleinsäuremolekülen detailliert erläutert.

"Ziel-DNA", so wie hier verwendet, bezeichnet das Genom des Aktinomyceten oder ein extrachromosomales Plasmid des Aktinomyceten, das modifiziert werden soll. Im erfindungsgemäßen Verfahren wird in einem ersten Schritt mindestens eine Erkennungssequenz in die Ziel-DNA eingefügt. Diese Erkennungssequenzen können von den Rekombinasen erkannt werden, woraufhin die Rekombinasen die Modifizierung der Ziel-DNA bewirken. Das Einfügen von Frt-, IoxP- und rox-Erkennungssequenzen kann durch Techniken erfolgen, die dem Fachmann bekannt sind (siehe Beispiele).

Eine Möglichkeit zum Einbringen von Erkennungssequenzen in das Genom der Wirtszelle ist in Abbildung 2 dargestellt. Hierbei wird zunächst ein Plasmid in den Mikroorganismus, hier vom Stamm *Streptomyces lividans,* eingebracht. Dieses Plasmid umfasst die Erkennungssequenzen und beispielsweise eine Bakteriophagenrekombinationsstelle (attP), die an die Region attB (attachment side on the bacterial chromosome) des Chromosoms der Wirtszelle binden kann. Es erfolgt eine Rekombination und das Nukleinsäuremolekül des Plasmids wird in das Genom der Wirtszelle integriert. Wie aus Abbildung 2 ersichtlich, sind nun zwei Frt-Erkennungssequenzen in der Ziel-DNA enthalten.

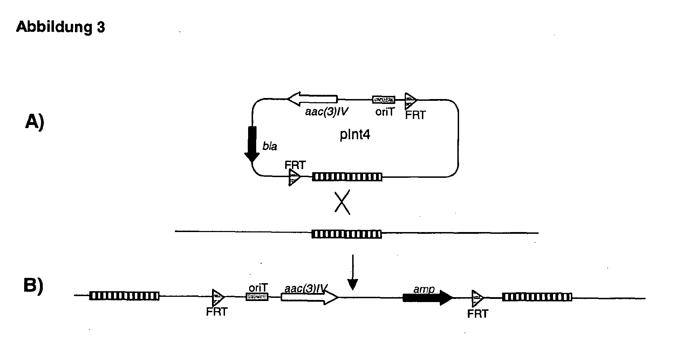

Ein weiteres Verfahren zum zielgerichteten Einbringen von Erkennungssequenzen ist die homologe Rekombination (Abbildung 3). Hierbei wird zunächst ein Plasmid in den Mikroorganismus, hier vom Stamm *Streptomyces species Tü6071* eingebracht. Dieses Plasmid umfasst beispielsweise Sequenzen, die homolog sind zu Sequenzen aus *Streptomyces species Tü6071.* Es erfolgt eine Rekombination und das Nukleinsäuremolekül des Plasmids wird in das Genom der Wirtszelle integriert. Wie aus Abbildung 3 ersichtlich, sind nun zwei Frt-Erkennungssequenzen in der Ziel-DNA enthalten.

Bevorzugterweise enthält die Ziel-DNA nach dem Einfügen von Erkennungssequenzen mindestens eine, weiter bevorzugt mindestens zwei, noch weiter bevorzugt mindestens drei und am meisten bevorzugt mindestens vier Erkennungssequenzen, die von einer Rekombinase erkannt werden können. Weiter bevorzugt ist es hierbei, dass die Ziel-DNA dann mindestens zwei Erkennungssequenzen aufweist, die jeweils von unterschiedlichen Rekombinasen erkannt werden können. Weiter bevorzugt werden mindestens drei, weiter bevorzugt mindestens vier, noch weiter bevorzugt mindestens fünf Erkennungssequenzen in die Ziel-DNA eingefügt, wobei mindestens zwei Erkennungssequenzen von unterschiedlichen Rekombinasen erkannt werden können.

Zur Durchführung von Modifizierungen der Ziel-DNA sind Rekombinasen notwendig. Diese werden durch Expression der erfindungsgemäßen Nukleinsäuremoleküle, die für diese Proteine kodieren, zur Verfügung gestellt. Diese Nukleinsäuremoleküle sind synthetische Gene, die durch Codon-usage-Optimierung hergestellt wurden. Hierbei wurden die von Aktinomyceten bevorzugten Codons verwendet. In Tabelle 1 sind die von Aktinomyceten bevorzugten Codons aufgeführt.

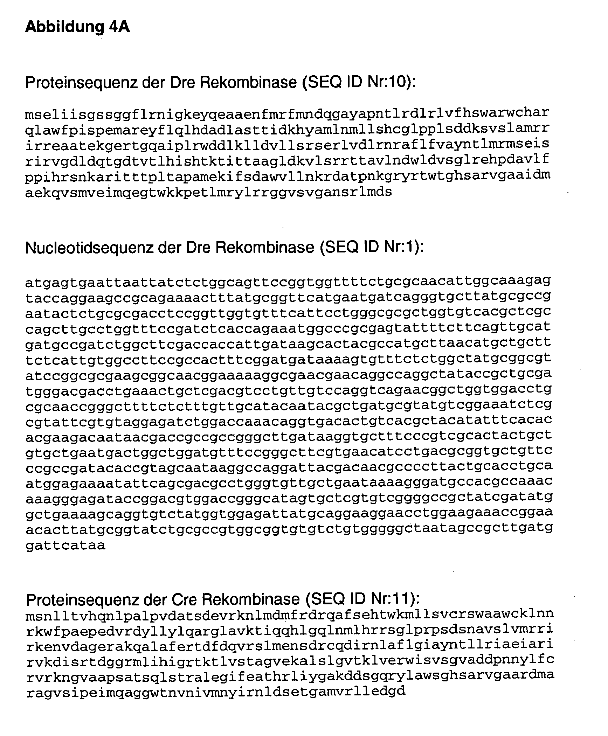

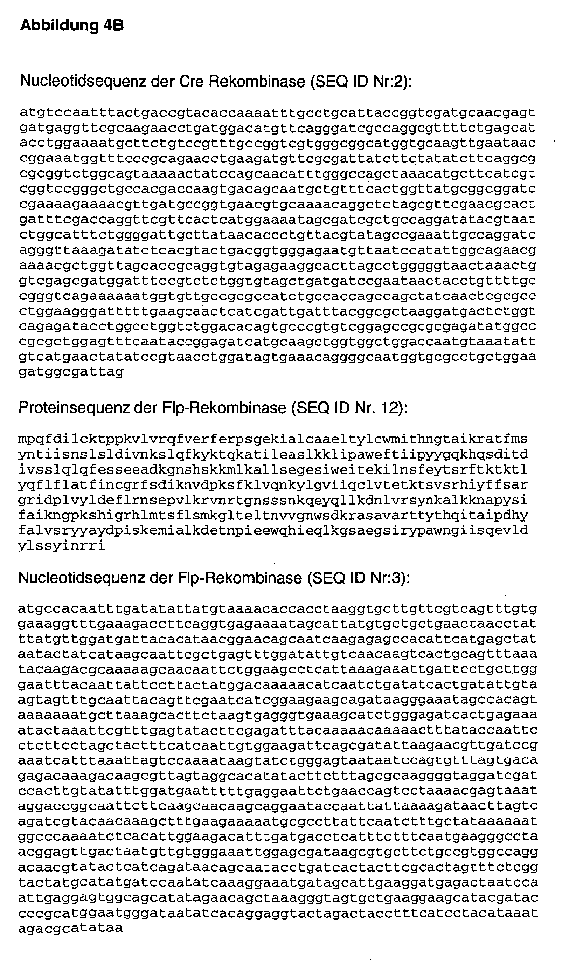

Um die synthetischen Gene zu erhalten, wurden die nicht von Aktinomyceten bevorzugten Triplets in den natürlichen Genen der Flp-, Cre- und Dre-Rekombinasen gegen Codons ausgetauscht, die von Aktinomyceten bevorzugt sind. Die natürlichen Nukleotidsequenzen der Dre-Rekombinase (SEQ ID Nr:1 , Genbank-Nr. AAV84949), der Cre-Rekombinase (SEQ ID Nr:2, Genbank-Nr. YP_006472), der Flp-Rekombinase (SEQ ID Nr:3, Genbank AAB59340) sind bekannt. In den Abbildungen 4A und 4B sind die Sequenzen der Rekombinasen und Rekombinasegene dargestellt (SEQ ID Nr:10-12).

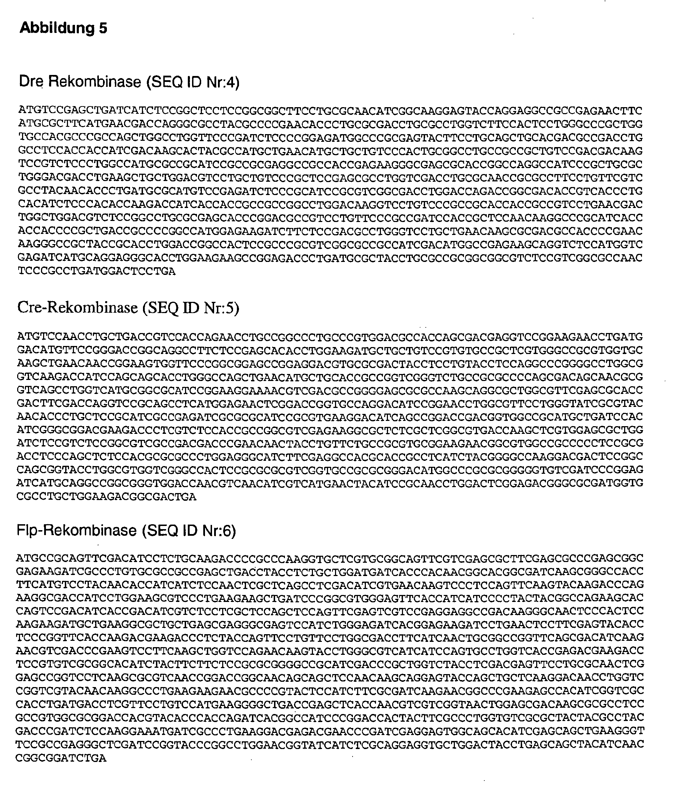

Durch Austausch aller von Aktinomyceten nicht bevorzugten Codons der natürlichen Nukleotidsequenzen gegen Codons, die gemäß Tabelle 1 von Aktinomyceten bevorzugt sind, wurden die erfindungsgemäßen Nukleotidsequenzen der Dre-Rekombinase (SEQ ID Nr:4), der Cre-Rekombinase (SEQ ID Nr:5), sowie der Flp-Rekombinase (SEQ ID Nr:6) erhalten. In Abbildung 5 sind die erfindungsgemäßen Sequenzen dargestellt.

Verfahren zur Herstellung von synthetischen, erfindungsgemäßen Nukleinsäuremolekülen mit den erfindungsgemäßen Nukleotidsequenzen sind dem Fachmann bekannt und in der Literatur ausführlich beschrieben (Hoover und Lubkowski, Nucleic Acids Res., 2002 30 (10), e43). Zur Ableitung der Primersequenzen wurde die Software "DNAWorks" (http://molbio.info.nih.gov/dnaworks) eingesetzt. Dieses Programm berechnet ausgehend von der gewünschten Aminosäuresequenz, den Daten aus einer artspezifischen Codongebrauchstabelle (Tabelle 1) und der gewünschten Länge und Schmelztemperatur der Oligonukleotide die "optimalen Primer".

Um das erfindungsgemäße Verfahren zur Modifizierung von Aktinomyceten durchführen zu können, ist es nicht notwendig, dass der Codon-Usage zu 100 % an die Aktinomyceten angepasst wird. Programme zur Erstellung von DNA-Sequenzen, die Codon-Usage optimiert sind, sind dem Fachmann bekannt (beispielsweise "Prot2DNA"). Die Synthese der berechneten Sequenzen kann beispielsweise unter Verwendung von Primern mit 20 Basenpaaren erfolgen. Die erfindungsgemäßen Nukleotidsequenzen weisen daher eine Identität von mindestens 80 %, weiter bevorzugt von mindestens 85 %, weiter bevorzugt von mindestens 90 %, noch weiter bevorzugt von mindestens 95 %, weiter bevorzugt von mindestens 98 % und am meisten bevorzugt von 100 % mit den Nukleotidsequenzen SEQ ID Nr.4-6 auf.

Der Ausdruck, dass eine DNA-Sequenz eine "Identität zu einer Nukleotidsequenz" aufweist, bedeutet, dass die zu vergleichenden Sequenzen, unter Verwendung eines üblichen Programms zum Vergleich von Nukleotidsequenzen, bei Standardparametern die spezifizierte prozentuale Übereinstimmung aufweisen.

Noch weiter bevorzugt weisen die Nukleinsäuremoleküle zu mindestens 70 % solche Codons auf, die von Aktinomyceten bevorzugt und in Tabelle 1 aufgeführt sind. Weiter bevorzugt weisen die Nukleinsäuremoleküle zu mindestens 75 %, weiter bevorzugt zu mindestens 80 %, noch weiter bevorzugt zu mindestens 85 %, noch weiter bevorzugt zu mindestens 90 % und am meisten bevorzugt zu mindestens 95 % von Aktinomyceten bevorzugte Codons aus Tabelle 1 auf.

Die Transformation (Aufnahme von DNA in prokaryotische Zellen) der Wirtszellen erfolgt mit den erfindungsgemäßen Nukleinsäuremolekülen, den einzufügenden DNA-Fragmenten sowie den Erkennungssequenzen bevorzugt unter Verwendung von Plasmiden. Hierbei können einerseits Plasmide verwendet werden, die die Nukleotidsequenzen enthalten, die für die Rekombinasen kodieren. Des Weiteren können Plasmide verwendet werden, um Erkennungssequenzen oder DNA-Fragmente in das Genom der Wirtszelle einzufügen.

Diese Plasmide tragen bevorzugt Gene die es ermöglichen, DNA-Fragmente in Aktinomyceten einzubringen: Bei replizierenden Vektoren (Beispiel: pUWL-T): ori-Rep (*E.coli*), Replikationsursprung; ori-Rep (*Actinomycet*)*,* Relikationsursprung; Antibiotika-Resistenzgen (*E.coli*); Antibiotika-Resistenzgen (*Actinomycet*); oriT - Sequenz notwendig für die Konjugation von *E.coli* zu den Actinomyceten (dies wird bei Transformationen nicht benötigt (dann Verwendung von pUWL201 möglich).

Bei integrierenden Vektoren: (Beispiel: plnt): intϕC31 - Integrase kodierendes Gen des Phagen ϕC31 notwendig für die Integration des Vektors in das Genom; attP, Erkennungssequenz des Plagen C31 notwendig für die Integration des Vektors in das Genom; ori-Rep (*E.coli*), Relikationsursprung; Antibiotika-Resistenzgen (*E.coli*); Antibiotika-Resistenzgen (*Actinomycet*); oriT - Sequenz notwendig für die Konjugation von *E.coli* zu den Actinomyceten (Bierman et al., Gene 1992, 116, 43).

Dies geschieht mit der sogenannten Konjugation, bei der ein Plasmid von einem *E. coli-*Stamm in einen Streptomyceten eingebracht wird. Die erfindungsgemäßen Nukleotidsequenzen liegen hierbei im Plasmid zusätzlich bevorzugt hinter einem ErmE-Promotor, der einen konstitutiven Promotor darstellt. Dadurch wird das Gen ständig exprimiert. Zusätzlich bevorzugt umfasst das Plasmid einen Replikationsursprung für Aktinomyceten. Zusätzlich bevorzugt enthält es auch einen Replikationsursprung für die Replikation in *E. coli.* Zusätzlich bevorzugt enthält das Plasmid auch ein Antibiotikum-Resistenzgen wie z.B. das Thiostreptonresistenzgen (tsr), das Apramycinresistenzgen (apr), das Hygromycinresistenzgen (hyg). Zusätzlich bevorzugt enthält das Plasmid auch einen induzierbaren Promoter (z.B. einen mit Thiostrepton induzierbaren Promoter) (Takano et al., Gene 1995, 166, 133.)

Geeignete Plasmide sowie notwendige Promotoren, Replikationsursprünge usw. sind dem Fachmann bekannt und können von diesem für die Transformation bzw. Konjugation von Aktinomyceten ausgewählt werden. Besonders bevorzugte Plasmide, die erfindungsgemäß verwendet werden können, sind pUWL201, pSET152, pKC1218 etc. (Bierman et al., Gene 1992, 116, 43.)

Erfindungsgemäß bevorzugte Plasmide sind pUWL-T, pUWL-A, pUWL-H, pNL1, pML1 und pAL1. Ein Plasmid kann aber durchaus ein, zwei oder drei Gene enthalten, die für unterschiedliche Rekombinasen kodieren). Mit den erfindungsgemäßen Rekombinationssystemen ist das Ausschneiden, Integrieren, Invertieren oder Translokieren von DNA-Fragmenten möglich.

Die Integration der Erkennungssequenzen werden mit Plasmiden durchgeführt, die keinen ori-Rep (Replikationsursprung Aktinomyceten), keine intϕC31-Gen und keine attP-Sequenz enthalten. Gene, die für Rekombinasen kodieren, werden mit replizierbaren Plasmiden, die keine intϕC31-Gen und keine attP-Sequenz enthalten, in die Stämme gebracht oder sie werden mit integrativen Vektoren, die ein intϕC31-Gen und eine attP-Sequenz enthalten, und einen induzierbaren Promoter enthalten, in die Stämme gebracht.

Der Begriff "Ausschneiden" von DNA-Fragmenten bedeutet, dass die Nukleotidsequenz zwischen zwei Erkennungssequenzen der Ziel-DNA von den Rekombinasen herausgeschnitten wird.

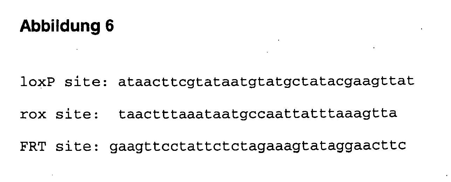

Die geeignete Erkennungssequenz für die Flp-Rekombinase ist die Frt-Erkennungssequenz mit der SEQ ID Nr.7. Die Erkennungssequenz für die Cre-Rekombinase ist die IoxP-Erkennungssequenz mit der SEQ ID Nr.8, die Erkennungssequenz der Dre-Rekombinase ist die rox-Erkennungssequenz mit der SEQ ID Nr.9. Die Synthese der Erkennungssequenzen bzw. der entsprechenden Nukleinsäuremoleküle kann nach üblichen Verfahren erfolgen und ist dem Fachmann bekannt (Hunkapiller et al., Nature 1984, 310, 105.) (Abbildung 6.)

Der Ausdruck "Integrieren" bedeutet, dass das einzufügende DNA-Fragment in einem Nukleinsäuremolekül zu einer Erkennungssequenz benachbart ist und von der Rekombinase in die Ziel-DNA integriert wird, wobei die Ziel-DNA ebenfalls diese Erkennungssequenz aufweist.
Der Ausdruck "Invertieren" bedeutet, dass das zu invertierende DNA-Fragment in einem Nukleinsäuremolekül zu einer Erkennungssequenz benachbart ist und von der Rekombinase invertiert, also umgedreht wird. Damit ändert sich die Orientierung des DNA-Fragments.

Der Ausdruck "Translozieren" bedeutet, dass ein DNA-Fragment auf der Ziel-DNA benachbart zu einer Erkennungssequenz, die Position mit einem weiteren DNA-Fragment auf der selben Ziel-DNA, ebenfalls benachbart zu einer Erkennungssequenz, tauscht.

Der Ausdruck "einzufügende DNA-Fragmente", so wie hier verwendet, umfasst jegliche DNA-Sequenzen unabhängig von ihrer Basenpaaranzahl. Analog dazu kann jedes Nukleinsäuremolekül bzw. jedes DNA-Fragment zwischen zwei Erkennungssequenzen aus der Ziel-DNA ausgeschnitten werden. Das einzufügende DNA-Fragment kann bevorzugt mittels eines Plasmids in die Wirtszelle eingebracht werden. Das Plasmid weist hierbei zusätzlich bevorzugt eine Erkennungssequenz auf, die einer Erkennungssequenz in der Ziel-DNA entspricht. Des Weiteren enthält das Plasmid zusätzlich bevorzugt einen Replikationsursprung für Aktinomyceten und weiter bevorzugt einen Replikationsursprung für *E. coli.*

Der Ausdruck "Wirtszellen" sowie "Wirtsorganismus" bezeichnet Zellen, die in der Lage sind die erfindungsgemäßen Nukleinsäuremoleküle zu exprimieren. Dies sind alle Mikroorganismen, bei denen die gleichen Codons wie bei den Aktinomyceten bevorzugt werden (siehe Tabelle 1). Insbesondere umfasst der Ausdruck "Wirtszellen" bzw. "Wirtsorganismus" daher bevorzugt Aktinomyceten, weiter bevorzugt Streptomyceten (als Gattung der Aktinomyceten), weiter bevorzugt die spezifischen Stämme *Streptomyces lividans,* Streptomyces coelicolor, Streptomyces albus, *Streptomyces cinnamonensis, Actinoplanes* sp. SE50/110, *Streptomyces galilaeus, Streptomyces nodosus, Actinosynnema pretiosum subsp. Auranticum, Streptomyces virido-chromogenes* Tü57, *Streptomyces echinatus, Streptomyces avermitilis* MA-4680, *Micromonospora echinospora, Streptomyces sp. FR-008, Streptomyces chartreusis, Streptomyces bikiniensis, Streptomyces roseochromogenes subsp. oscitans* DS12.976, *Streptomyces griseus* subsp. griseus, *Streptomyces neyagawaensis, Streptomyces olindensis, Streptomyces rishiriensis* DSM 40489, *Streptomyces venezuelae, Streptomyces peucetius, Streptomyces olivaceus* Tü2353, *Saccharopolyspora erythraea, Micromonospora carbonacea* var. Africana ATCC39149, *Streptomyces griseoflavus* Gö3592, *Streptomyces ambofaciens, Streptomyces violaceoruber* Tü22, *Amycolatopsis orientalis, Amycolatopsis orientalis, Amycolatopsis mediterranei (balhimycina)* DSM5908, *Micromonospora echinospora* ATCC15835, *Streptomyces griseoruber, Streptomyces aureofaciaens* Tü11, *Streptomyces antibioticus* Tü99, *Streptomyces antibioticus* Tü1718, *Streptomyces sp.* Tü2187, *Streptomyces sp.* Tü1156, *Streptomyces sp.* FU107, *Streptomyces sp.* FU36, *Streptomyces venezuelae* ISP5230, *Streptomyces kanamyceticus, Streptomyces cyanogenus* S136, *Streptomyces aureofaciens* Tü117, *Streptomyces rochei, Streptomyces globisporus* 1912, *Streptomyces* sp. AM-7161, *Micromonospora megalomicea, Streptomyces argillaceus, Streptomyces carzinostaticus* ATCC 15944, *Streptomyces fradiae* NCIB 8233, *Streptomyces spheroides,NCIMB* 11891, *Streptomyces noursei* ATCC11455, *Streptomyces antibioticus, Actinoplanes teichomyceticus, Streptomyces natalensis, Streptomyces* sp. Tü 6071, *Streptomyces diastato-chromogenes* Tü6028, *Lechevalieria aerocolonigenes* ATCC39243, *Saccharothrix espanaensis, Micromonospora* sp. Tü 6368, *Streptomyces antibioticus* Tü6040, *Streptomyces nogalater, Sorangium cellulosum* So ce12, *Saccharopolyspora, Spinosa, Streptomyces* sp. TP-A0274, *Streptomyces steffisburgensis* NRRL 3193, *Streptomyces griseus, Streptomyces fradice, Streptomyces fradiae* Tü2717, und *Streptomyces halstedii* HC-34.

Das erfindungsgemäße Verfahren zur Herstellung von modifizierten Mikroorganismen umfasst das Einfügen von mindestens einer Erkennungssequenz, bevorzugt von zwei Erkennungssequenzen, in das Genom (Ziel-DNA) des Aktinomyceten (Schritt (a)), das Einbringen mindestens eines Plasmids mit dem erfindungsgemäßen Nukleinsäuremolekül in den Aktinomyceten (Schritt (b)), sowie das Vervielfältigen des Plasmids und Expression der Rekombinasen, die die Ziel-DNA modifiziert/modifizieren (Schritt (c)).

Schritt (a): In diesem Schritt werden Erkennungssequenzen in die Ziel-DNA eingefügt, wobei die Erkennungssequenzen ausgewählt sind aus der Gruppe bestehend aus Frt-, IoxP- und rox-Erkennungssequenzen. Das Einfügen der Erkennungssequenzen erfolgt bevorzugterweise unter Verwendung von Plasmiden. Das Einfügen der Nukleinsäuremoleküle, die die Erkennungssequenzen enthalten, kann beispielsweise über homologe Rekombination unter Verwendung homolger DNA erfolgen, die bevorzugterweise ebenfalls im Plasmid enthalten ist. Zusätzlich bevorzugt enthält das Plasmid des Weiteren notwendige Promotoren und Replikationsursprünge für *E. coli*-Stämme.

Schritt (b): In einem anschließenden Schritt werden die erfindungsgemäßen Nukleinsäuremoleküle in die Aktinomyceten eingebracht. Bevorzugterweise erfolgt dies ebenfalls unter Verwendung von Plasmiden. Das Einbringen der Plasmide in die Aktinomyceten erfolgt wie in Schritt (a) mittels Transformation (bzw. Kojugation). Bevorzugterweise umfassen diese Plasmide die notwendigen Promotoren und Replikationsursprünge für Aktinomyceten und *E. coli*-Stämme, weiter bevorzugt für Aktinomyceten.

Schritt (c): In einem anschließenden Schritt wird das Plasmid aus Schritt (b) in dem Aktinomyceten vervielfältigt. Das Vervielfältigen der Plasmide in den Schritten (a) und (b) kann unter Verwendung üblicher Bedingungen erreicht werden. Des Weiteren werden die Plasmide in den Aktinomyceten exprimiert bzw. die Rekombinasen dadurch hergestellt. Die Rekombinasen erkennen dann die Erkennungssequenzen in der Ziel-DNA und bewirken ortsspezifische Veränderungen der Ziel-DNA.

Wird in Schritt (c) des obigen Verfahrens mindestens ein DNA-Fragment in die Ziel-DNA eingefügt, so wird hierzu zusätzlich bevorzugt ein weiteres Plasmid verwendet, das das einzufügende DNA-Fragment und eine Erkennungssequenz enthält, wobei die Erkennungssequenz der Ziel-DNA entspricht. Werden mehr als ein DNA-Fragment in die Ziel-DNA eingefügt, so können dazu mehrere Plasmide verwendet werden. Bevorzugt enthalten diese Plasmide zusätzlich die notwendigen Promotoren und Replikationsursprünge für die Vervielfältigung in Aktinomyceten.

Die Bedingungen zur Transformation (bzw. Konjugation) von Wirtszellen sind dem Fachmann aus der Literatur bekannt (Kieser et al., Practical Streptomyces Genetics, The John Innes Foundation, Norwich, 2000).

Für das Ausschneiden von DNA-Fragmenten ist das Vorhandensein von mindestens zwei gleichen Erkennungssequenzen für eine spezifische Rekombinase notwendig.

Bevorzugterweise werden beim Einfügen von mehr als einem DNA-Fragment verschiedene Rekombinationssysteme bzw. Rekombinasen verwendet. Hierbei werden bevorzugt mindestens zwei verschiedene Erkennungssequenzen, ausgewählt aus den Frt-, IoxP- und rox-Rekombinationserkennungssequenzen in die Ziel-DNA eingefügt. Dies ermöglicht beispielsweise das anschließende Einfügen von zwei verschiedenen DNA-Fragmenten, die zu unterschiedlichen Erkennungssequenzen benachbart sind, wobei beispielsweise ein DNA-Fragment unter Verwendung der Frt-Erkennungssequenz und der Flp-Rekombinase und das andere DNA-Fragment beispielsweise unter Verwendung der IoxP-Erkennungssequenz und der Cre-Rekombinase eingefügt werden kann. Das Verfahren zur Modifizierung von Mikroorganismen ist in den Beispielen weiter ausgeführt.

Zusätzlich ist es bevorzugt, dass ein Markergen zur Identifizierung und Selektion der modifizierten Wirtszellen ebenfalls in die Ziel-DNA eingebracht wird. Derartige Markergene sind dem Fachmann bekannt und in der Literatur beschrieben (DeWet et al., Mol. Cell. Biol., 1987, 7, 725-737; Goff et al., EMBO J., 1990, 9, 2517-2522; Kain et al., BioTechniques, 1995, 19, 650-655; Chiu et al., Current Biology, 1996, 6, 325-330). Geeignete Markergene sind beispielsweise Gene die Antibiotikaresistenz vermitteln, wie beispielsweise Apramycin, Thiostrepton, Chloramphenicol, Methodrexate, Hygromycin, Streptomycin, Spectinomycin oder Bleomycin (Herrera Estrella et al., EMBO, J. 1983, 2, 987-992; Herrera Estrella et al., Nature, 1983, 303, 209-213; Meijer et al., Plant. Mol. Biol., 1991, 16, 807-820; Waldron et al., Plant Mol. Biol., 1985, 5, 103-108; Zhijian et al., Plant Science, 1995; 108, 219-227; Jones et al., Mol. Gen. Genet., 1987, 210, 86-91; Bretagne-Sagnard et al., Transgenic Res., 1996, 5. 131-137; Hille et al., Plant Mol. Biol., 1990, 7, 171-176; Guerineau et al., Plant Mol. Biol., 1990, 15, 127-136).

Das erfindungsgemäße Rekombinationssystem der vorliegenden Erfindung zum Modifizieren von Mikroorganismen umfasst mindestens ein erfindungsgemäßes Nukleinsäuremolekül, das für eine Rekombinase kodiert. Des Weiteren enthält es eine Ziel-DNA, die mindestens eine Erkennungssequenz aufweist. Für das Rekombinationssystem können alle im Vorangegangenen beschriebenen erfindungsgemäßen Nukleinsäuremoleküle, Plasmide und jede Ziel-DNA verwendet werden. Bevorzugt werden Apramycin oder Hygromycin als Antibiotika verwendet.

Der erfindungsgemäße, modifizierte Mikroorganismus kann unter Verwendung der hier beschriebenen Verfahren hergestellt werden. Aus einem bekannten Überproduktionsstamm, der einen bestimmten Naturstoff in großen Mengen herstellen kann, wird mit der neuen Technologie das Biosynthesegencluster (Biosynthesegencluster I) für den Naturstoff entfernt. Das gesamte Biosynthesegencluster (Biosynthesegencluster II) eines Naturstoffes, dessen Synthese in großen Mengen erwünscht ist, wird ebenfalls mit der neuen Technologie kloniert und anschließend in den veränderten Überproduktionsstamm gebracht. Es kann möglich sein, zuvor Promotoren auf dem Biosynthesegencluster II auszutauschen. Somit überträgt man die Syntheseleistung für den Naturstoff I auf die Syntheseleistung für den Naturstoff II.

**Abbildung 1** betrifft Cre-Iox-Reaktionen in Abhängigkeit der Orientierung und räumlichen Anordnung der IoxP-Schnittstellen. Gepaarte IoxP-Erkennungssequenzen (Dreiecke) sind richtungsabhängig und können in cis- (gleicher DNA-Strang) oder trans- (verschiedene DNA-Stränge) Anordnung lokalisiert werden.
(A) Falls die IoxP-Erkennungssequenzen ein DNA Segment in Cis-Anordnung flankieren und in der gleichen Richtung orientiert sind, vermittelt Cre-Rekombination das Ausschneiden und die Zirkularisierung des Segmentes.
(B) Falls die IoxP-Erkennungssequenzen ein DNA Segment in Cis-Anordnung flankieren und in entgegengesetzter Richtung orientiert sind, vermittelt Cre-Rekombination eine Inversion des Segmentes.
(C) Falls die IoxP-Erkennungssequenzen auf verschiedenen DNA-Strängen lokalisiert und in der selben Richtung orientiert sind, vermittelt Cre-Rekombination eine Verschiebung des Segmentes auf den jeweils anderen Strang.

**Abbildung 2** zeigt ein Schema zur Integration von plnt3 in das Chromosom von *S. lividans* ((A) und (B)). FRT, Erkennungssequenz der Flp-Rekombinase; aac(3)IV, Apramycin-Resistenzgen; oriT, Sequenz notwendig für die Konjugation von *E.coli* zu den Actinomyceten; bla, β-Lactamasegen; intϕC31, Gen, das für die Integrase des Phagen ϕC31 kodiert und für die Integration von plnt3 in das Chromosom von *S. lividans* verantwortlich ist; attP, Bindestelle des Phagen ϕC31; attB, Bindestelle im Chromosom von *S. lividans;* P1 und P2, Primerbindestellen zur Detektion der von FRT-Erkennungssequenzen umgebenen DNA.

**Abbildung 3** zeigt das Schema zur Integration von plnt4 in das Chromosom von *Streptomyces species Tü6071* mittels homologer Rekombination. FRT, Erkennungssequenz der Flp-Rekombinase; aac(3)IV, Apramycin-Resistenzgen; oriT, Sequenz notwendig für die Konjugation von *E.coli* zu den Actinomyceten; bla, β-Lactamasegen; gestrichelte Box, homologe DNA-Bereiche.

**Abbildungen 4A und 4B** zeigen die Originalsequenzen (Gensequenzen SEQ ID Nr:1-3 und Proteinsequenzen SEQ ID Nr:10-12) der Rekombinasen Cre, Dre und Flp.

**Abbildung 5** zeigt die erfindungsgemäßen Gensequenzen der Rekombinasen, Dre (SEQ ID Nr:4), Cre (SEQ ID Nr:5) und Flp (SEQ ID Nr:6).

**Abbildung 6** zeigt die Erkennungssequenzen Frt (SEQ ID Nr:7), IoxP (SEQ ID Nr:8) und rox (SEQ ID Nr:9).

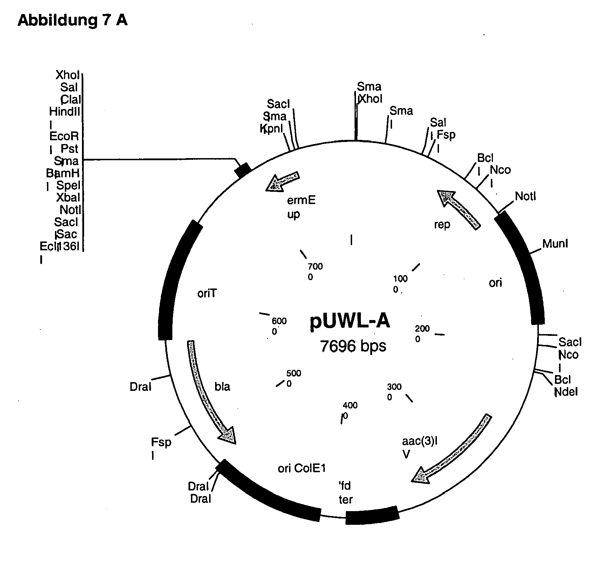

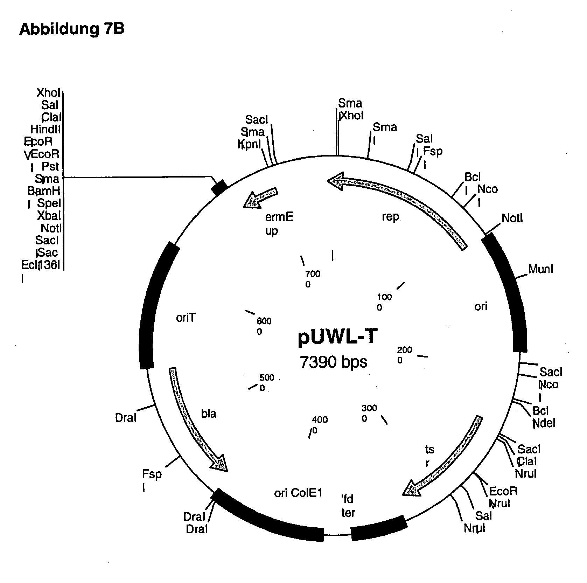

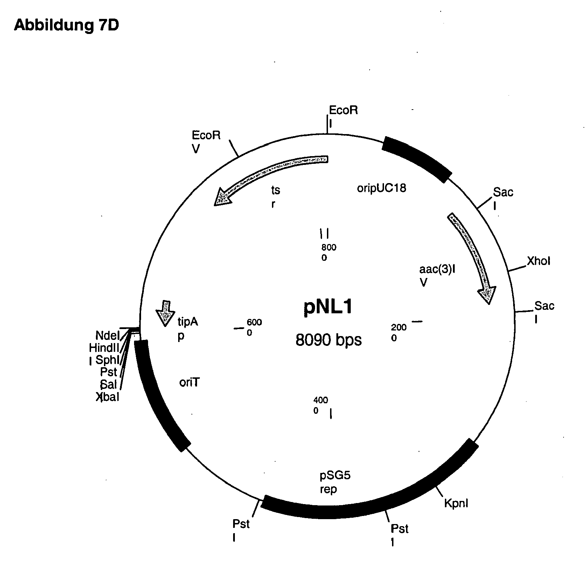

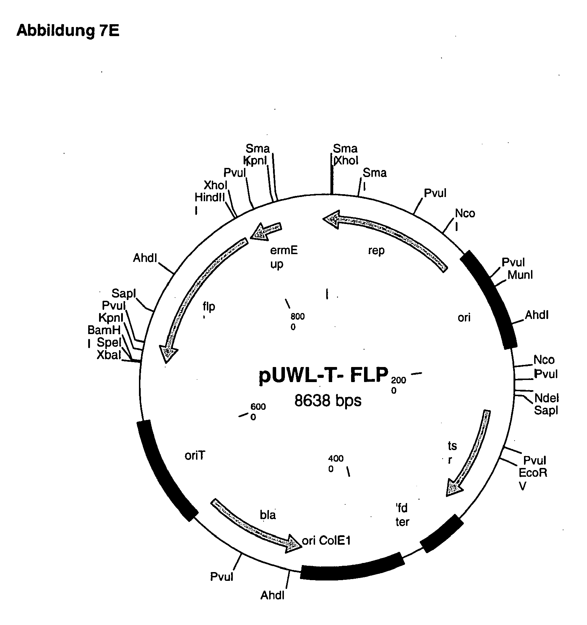

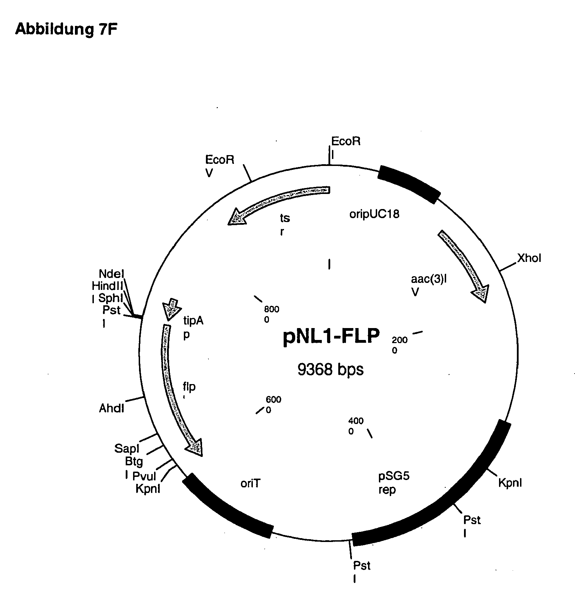

**Abbildungen 7A-F** zeigen die erfindungsgemäßen Plasmide pUWL-T, pUWL-A, pUWL-H, und pNL1, die man einsetzen kann, um die Rekombinasen in Aktinomyceten einzubringen und zu exprimieren und beispielsweise die Plasmide pUWL-T-FLP und pNL1-FLP. pUWL-T wurde erhalten aus pUW201 durch einbringen des oriT (Sequenz wichtig für den Konjugationsvorgang). pUWL-A und pUWL-H entstanden aus pUWL-T durch Austausch der Antibiotikaresistenzgene (T, Thiostrepton; A, Apramycin, H, Hygromycin). pUWL-T-FLP entstand aus pUWL-T durch einklonieren des FLP-Rekombinasegens in die *BamHI* und *HindIII* Schnittstellen des Vektors pUWL-T. pNL1 entstand aus pIJ8600 (Klonierung eines *Ecol*RI-*Bam*HI-Fragmentes, das den tipA Promotor und das Thiostreptonresistenzgen enthält) und pKC1139 (Klonierung eines *Ecol*RI-*Bgl*II-Fragmentes, das das temperatursensitive Replikon pSG5 und das Apramycinrseistenzgen aac(3)IV enthält). pNL1-FLP entstand aus pNL1 durch Einklonieren des FLP-Rekombinasegens in die *Spel* und *Xbal* Schnittstellen des Vektors pNL1.

### Verwendete Abkürzungen:

Nukleotidsequenz flp', die für die Flp-Rekombinase kodiert. Weitere Abkürzungen: *ErmE,* starker Promotor; bla, β-Lactamase; ori ColE1, Replikationsursprung notwendig für die Replikation in *E. coli*; ori pUC18, Replikationsursprung notwendig für die Replikation in *E. coli*; tsr, Thiostreptonresistenzgen; aac(3)IV, Apramycinrseistenzgen, hyg, Hygromycinresistenzgen, rep^{PIJ101}, Replikationsursprung notwendig für die Replikation in Aktinomyceten. pSG5 rep, temperatursensitiver Replikationsursprung notwendig für die Replikation in Aktinomyceten, tipA, Promotor;

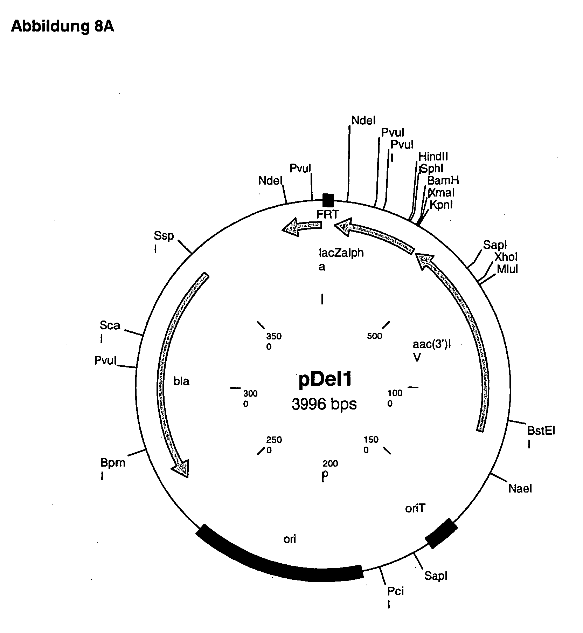

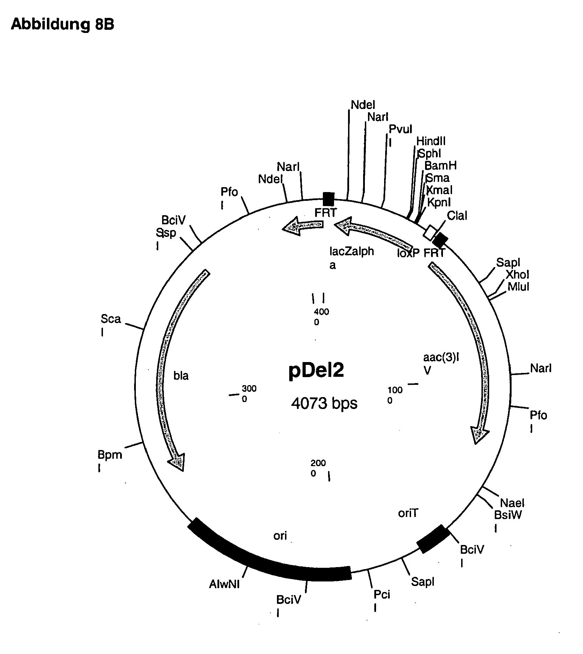

**Abbildungen 8A und B** zeigen die erfindungsgemäßen Plasmide, die man einsetzen kann, um ein Naturstoffbiosynthesegencluster zu deletieren und zu klonieren.
Plasmid pDel1 und pDel2 wurden durch PCR-Fusion hergestellt. Ausgangsvektoren für die PCR waren pUC19 und plJ773 (Khodakaramian et al., Nucleic Acids Res., 2006, 34, e20).

### Verwendete Abkürungen:

FRT, Erkennungssequenz der Flp-Rekombinase; carb, β-Lactamasegen; oriT, Sequenz notwendig für die Konjugation von *E.coli* zu den Actinomyceten; ori, Sequenz notwendig für die Replikation in *E. coli;* lacZ und lacZ', Sequenzen des β-Galaktosidasegens; IoxP, Erkennungssequenz der Cre-Rekombinase (zusätzliches DNA-Element, das derzeit nicht benötigt wird); apra, Apramycin-Resistenzgen. Abbildung 4b zeigt die Generierung einer Mutante des Phenalinolactom-Produzenten bei gleichzeitiger Klonierung des Phenalinolactombiosynthesegenclusters. Die Frt-Erkennungssequenzen sind als Dreiecke dargestellt. Am^{r}, Apramycinresistenzgen; ori^{pUC}, Replikationsursprung, notwendig für die Replikation in *E. coli.*

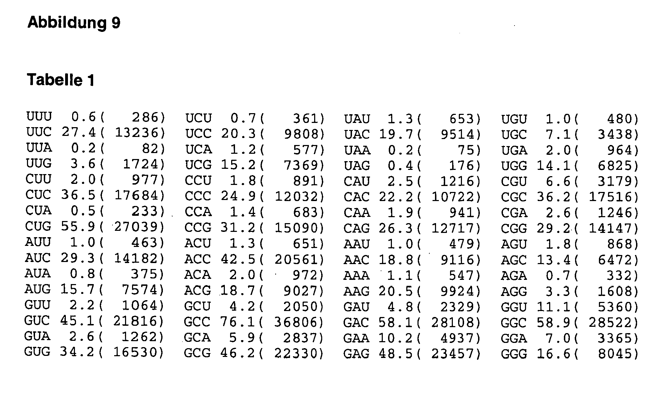

**Abbildung 9** zeigt Tabelle 1. Tabelle 1 zeigt den Codongebrauch von Aktinomyceten. (http://www.nih.go.jp/∼jun/act/codon/104.html).

### Beispiel 1 - Synthese der erfindungsgemäßen Nukleinsäuremoleküle

Die Synthese der Moleküle wurden von der Firma DNA 2.0 (FLP und Cre) und der Firma Genscript (Dre) durchgeführt.

### Beispiel 2 - Synthese der Plasmide mit den erfindungsgemäßen Nukleinsäuremolekülen

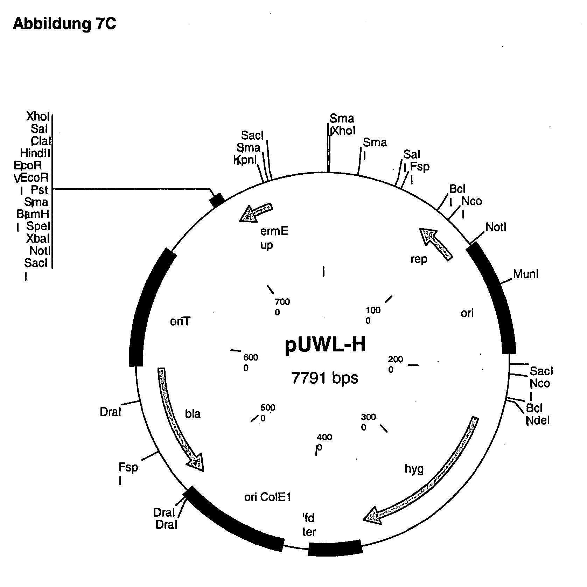

Um das Flp-Gen in die Wirtszelle einzubringen und dort zu exprimieren, wurde das Plasmid pUWL-H verwendet. Das künstliche Flp-Gen wurde in dem Plasmid direkt hinter einem ErmE Promotor, einem konstitutiven Promotor einkloniert. Dadurch wird das Gen ständig exprimiert (siehe auch Abbildung 7C)

### Beispiel 3 - Einfügen von Erkennungssequenzen in die Ziel-DNA

Plasmide können mittels Konjugation oder Transformation in die Aktinomycetenstämme eingebracht werden. Versuchsbeschreibung finden sich in Kieser et al. (Kieser et al., Practical Streptomyces Genetics, The John Innes Foundation, Norwich, 2000).

### Beispiel 4 - Induzierung der Rekombination

Die Induktion der Rekombinase, die hinter den tipA-Promoter kloniert wurde, erfolgt durch Zugabe von Thiostrepton (3.5 µg/ml).

### Beispiel 5 - Einfügen und Ausschneiden einer Apramycinresistenzkassette

In diesem Experiment wurde eine Apramycinresistenzkassette, die von Frt-Erkennungssequenzen flankiert ist in den Stamm *Streptomyces lividans* TK24 eingefügt und dann wieder ausgeschnitten. Hierzu wurde das in Beispiel 7 beschriebene Plasmid mit dem erfindungsgemäßen Flp-Gen verwendet. Des Weiteren wurde ein Plasmid verwendet, das die einzufügende Apramycinresistenzkassette flankiert von Frt-Erkennungssequenzen enthält. Das Plasmid enthält außerdem ein intϕC31-Gen, das für die Integrase des Phagen ϕC31 kodiert und für die Integration von plnt3 in das Chromosom von *S. lividans* verantwortlich ist sowie zudem eine attP-Bindestelle des Phagen ϕC31.

Durch die Integration des Plasmids wurde der Streptomyces-Stamm gegen Apramycin resistent. Ebenfalls per Konjugation wurde das Plasmid pUWLFLP in den gleichen Stamm eingebracht. Durch die aktiven Flp-Gene wurde in einigen Kolonien/Klonen die Apramycinresistenzkassette aus dem Genom des Streptomyceten wieder ausgeschnitten. 40 % (56 Kolonien) der 140 untersuchten Klone waren sensitiv gegen Apramycin. Dies bedeutet, dass das Frt/Flp-System erfolgreich in Aktinomyceten angewendet werden kann. Um dies weiter zu belegen, wurde die DNA der 56 Klone isoliert und mit spezifischen Primern für das Apramycinresistenzgen eine PCR durchgeführt. Da kein PCR-Produkt nachgewiesen werden konnte, wurde gezeigt, dass das Gen erfolgreich durch die Flp-Rekombinase entfernt worden ist.

### Beispiel 6 - Herstellen eines Überproduktionsstammes

Zur Herstellung eines Überproduktionsstammes nimmt man einen Aktinomycetenstamm, der große Mengen eines Naturstoffs produziert. Aus diesem Stamm wird das Biosynthesegencluster mit den beschriebenen Methoden entfernt. Biosynthesegencluster der Naturstoffe, die nun in großen Mengen produziert werden sollen, werden in die generierte Mutante gebracht. Denkbar ist, dass die Promotoren auf den einzubringenden Clustern durch Promotoren auf dem ursprünglichen Cluster ersetzt werden müssen.
Die Produktion großer Mengen eines Naturstoffs wird dann gelingen, wenn die Biosyntheseausgangsverbindungen des ursprünglichen Naturstoffs und des neuen Naturstoffs identisch sind.

### Beispiel 7 - Rekombinase katalysierter Genaustausch

Es ist auch möglich, einen Rekombinase katalysierten Genaustausch durchzuführen. Dieses Verfahren ist in der Literatur als RMCE (recombinase-mediated-cassette exchange) bekannt. Zunächst werden die Erkennungssequenzen FRT und F₃ (homotypische und heterotypische Sequenzen) sowohl in das Genom eines Stammes als auch in ein Plasmid einkloniert. Die Erkennungssequenzen werden "upstream" und "downstream" des austauschenden Gens bzw. "upstream" und "downstream" des einzubringenden Gens gebracht. Nach Expression der FLP-Rekombinase wird das "alte" Gen durch das "neue" ersetzt. Mit diesem Verfahren können auch ganze Gencluster kloniert werden.

## Patentansprüche

1. In Wirtszellen replizierbares Nukleinsäuremolekül, enthaltend mindestens eine Nukleinsäuresequenz, ausgewählt aus der Gruppe bestehend aus:
i) DNA-Sequenzen, die über die ganze Länge mindestens 80% Identität zu der Nukleotidsequenz SEQ ID Nr. 4 aufweisen und für eine Dre-Rekombinase kodieren,
ii) DNA-Sequenzen, die über die ganze Länge mindestens 80% Identität zu der Nukleotidsequenz SEQ ID Nr. 5 aufweisen und für eine Cre-Rekombinase kodieren,
iii) DNA-Sequenzen, die über die ganze Länge mindestens 80% Identität zu der Nukleotidsequenz SEQ ID Nr. 6 aufweisen und für eine FLP-Rekombinase kodieren.

2. In Wirtszellen replizierbares Nukleinsäuremolekül gemäß Anspruch 1, **dadurch gekennzeichnet, dass** die Nukleotidtrippletts aus der Gruppe i) - iii) zu mindestens 70% solche Codons darstellen, die von Aktinomyceten bevorzugt sind.

3. In Wirtszellen replizierbares Nukleinsäuremolekül nach einem der vorangegangenen Ansprüche, **dadurch gekennzeichnet, dass** es in einem *Escherichia coli-* oder *Aktinomyceten-Stamm* verwendet werden kann.

4. In Wirtszellen replizierbares Nukleinsäuremolekül nach einem der vorangegangenen Ansprüche, **dadurch gekennzeichnet, dass** es in einem *Streptomyceten* -Stamm vervielfältigt werden kann.

5. In Wirtszellen replizierbares Nukleinsäuremolekül nach Anspruch 4, **dadurch gekennzeichnet, dass** der Wirtszellen Streptomyceten-Stamm ausgewählt ist aus: *Streptomyces lividans,* Streptomyces coelicolor, Streptomyces albus, *Streptomyces cinnamonensis, Actinoplanes* sp. SE50/110, *Streptomyces galilaeus, Streptomyces nodosus, Actinosynnema pretiosum subsp. Auranticum, Streptomyces virido-chromogenes* Tü57, *Streptomyces echinatus, Streptomyces avermitilis* MA-4680, *Micromonospora echinospora, Streptomyces sp. FR-008, Streptomyces chartreusis, Streptomyces bikiniensis, Streptomyces roseochromogenes subsp. oscitans* DS12.976, *Streptomyces griseus* subsp. griseus, *Streptomyces neyagawaensis, Streptomyces olindensis, Streptomyces rishiriensis* DSM 40489, *Streptomyces venezuelae, Streptomyces peucetius, Streptomyces olivaceus* Tü2353, *Saccharopolyspora erythraea, Micromonospora carbonacea* var. Africana ATCC39149, *Streptomyces griseoflavus* Gö3592, *Streptomyces ambofaciens, Streptomyces violaceoruber* Tü22, *Amycolatopsis orientalis, Amycolatopsis orientalis, Amycolatopsis mediterranei (balhimycina)* DSM5908, *Micromonospora echinospora* ATCC15835, *Streptomyces griseoruber, Streptomyces aureofaciaens* Tü11, *Streptomyces antibioticus* Tü99, *Streptomyces antibioticus* Tü1718, *Streptomyces sp.* Tü2187, *Streptomyces sp.* Tü1156, *Streptomyces sp.* FU107, *Streptomyces sp.* FU36, *Streptomyces venezuelae* ISP5230, *Streptomyces kanamyceticus, Streptomyces cyanogenus* S136, *Streptomyces aureofaciens* Tü117, *Streptomyces rochei, Streptomyces globisporus* 1912, *Streptomyces* sp. AM-7161, *Micromonospora megalomicea, Streptomyces argillaceus, Streptomyces carzinostaticus* ATCC 15944, *Streptomyces fradiae* NCIB 8233, *Streptomyces spheroides,* NCIMB 11891, *Streptomyces noursei* ATCC11455, *Streptomyces antibioticus, Actinoplanes teichomyceticus, Streptomyces natalensis, Streptomyces* sp. Tü 6071, *Streptomyces diastato-chromogenes* Tü6028, *Lechevalieria aerocolonigenes* ATCC39243, *Saccharothrix espanaensis, Micromonospora* sp. Tü 6368, *Streptomyces antibioticus* Tü6040, *Streptomyces nogalater, Sorangium cellulosum* So ce12, *Saccharopolyspora, Spinosa, Streptomyces* sp. TP-A0274, *Streptomyces steffisburgensis* NRRL 3193, *Streptomyces griseus, Streptomyces fradice, Streptomyces fradiae* Tü2717, *Streptomyces halstedii* HC-34.

6. In Wirtszellen replizierbares Nukleinsäuremolekül nach einem der vorangegangenen Ansprüche, **dadurch gekennzeichnet, dass** es ein Plasmid darstellt.

7. In Wirtszellen replizierbares Nukleinsäuremolekül nach einem der vorangegangenen Ansprüche, **dadurch gekennzeichnet, dass** das Plasmid einen ErmE- oder tipA-Promotor aufweist.

8. In Wirtszellen replizierbares Nukleinsäuremolekül nach einem der vorangegangenen Ansprüche, **dadurch gekennzeichnet, dass** das Plasmid ausgewählt ist aus der Gruppe bestehend aus: pUWL-T-FLP, pUWL-A-FLP, pUWL-H-FLP, pNL1-FLP, pML1-FLP und pAL1-FLP, pUWL-T-Dre, pUWL-A-Dre, pUWL-H-Dre, pNL1-Der, pML1-Dre und pAL1-Dre, pUWL-T-Cre, pUWL-A-Cre, pUWL-H-Cre, pNL1-Cre, pML1-Cre und pAL1-Cre.

9. Rekombinationssystem zum Ausschneiden, Integrieren, Invertieren oder Translozieren von DNA-Fragmenten aus/in dem/das Genom (Ziel-DNA) von Aktinomyceten, **dadurch gekennzeichnet, dass** es mindestens ein Nukleinsäuremolekül gemäß einem der Ansprüche 1 - 8 und bei Verwendung von Rekombinasen mindestens eine Erkennungssequenz in der Ziel-DNA aufweist.

10. Rekombinationssystem gemäß Anspruch 9, **dadurch gekennzeichnet, dass** die Erkennungssequenz ausgewählt ist aus der Gruppe bestehend aus: FRT-, IoxP-, und rox-Erkennungssequenzen.

11. Rekombinationssystem gemäß Anspruch 10, **dadurch gekennzeichnet, dass** die Ziel-DNA mindestens zwei Erkennungssequenzen, ausgewählt aus der Gruppe bestehend aus: FRT-, IoxP-, und rox-Erkennungssequenzen, enthält.

12. Verfahren zur Herstellung von modifizierten Aktinomyceten, umfassend die folgenden Schritte:
a) Einfügen von mindestens einer Erkennungssequenz in das Genom (Ziel-DNA) des Aktinomyceten, wobei die Erkennungssequenzen ausgewählt sind aus der Gruppe betsehend aus Frt-, IoxP- und rox-Erkennungssequenzen,
b) Einbringen mindestens eines Plasmids in den Aktinomyceten, wobei das Plasmid ein replizierbares Nukleinsäuremolekül gemäß einem der Ansprüche 1-8 darstellt, und
c) Vervielfältigen des Plasmids aus b) und Exprimieren der Rekombinase, die für die Erkennungssequenz(en) der Ziel-DNA spezifisch ist, ein DNA-Fragement der Ziel-DNA ausschneidet, translokiert, invertiert oder ein DNA-Fragment in die Ziel-DNA einfügt.

13. Verfahren gemäß Anspruch 12, **dadurch gekennzeichnet, dass** das Einfügen der Erkennungssequenzen in die Ziel-DNA unter Verwendung eines weiteren Plasmids erfolgt, wobei das Plasmid die Erkennungssequenz und eine attP-Erkennungssequenz enthält.

14. Verfahren gemäß einem der Ansprüche 12 oder 13, **dadurch gekennzeichnet, dass** mindestens zwei Erkennungssequenzen, ausgewählt aus der Gruppe bestehend aus: Frt-, IoxP-, und rox-Erkennungssequenzen in die Ziel-DNA eingebracht werden.

15. Verfahren gemäß einem der Ansprüche 12 - 14, **dadurch gekennzeichnet, dass** mindestens zwei unterschiedliche Nukleinsäuremoleküle gemäß einem der Ansprüche 1-8 verwendet werden.

16. Verwendung eines Rekombinationssystems gemäß einem der Ansprüche 9-11 zum Ausschneiden, Integrieren, Invertieren oder Translozieren von DNA-Fragmenten in Aktinomyceten.

17. Modifizierter Mikroorganismus, hergestellt durch ein Verfahren gemäß einem der Ansprüche 12 - 15.


SEQUENCE LISTING

<110> Albert-Ludwigs- Universität Freiburg

<120> Herstellung von genetisch modifizierten Aktinomyceten durch
      Rekombination

<130> Sequenzprotokoll

<160> 12

<170> PatentIn version 3.3

<210> 1
<211> 1029
<212> DNA
<213> enterobacteria phage D6

<400> 1
atgagtgaat taattatctc tggcagttcc ggtggttttc tgcgcaacat tggcaaagag      60

taccaggaag ccgcagaaaa ctttatgcgg ttcatgaatg atcagggtgc ttatgcgccg     120

aatactctgc gcgacctccg gttggtgttt cattcctggg cgcgctggtg tcacgctcgc     180

cagcttgcct ggtttccgat ctcaccagaa atggcccgcg agtattttct tcagttgcat     240

gatgccgatc tggcttcgac caccattgat aagcactacg ccatgcttaa catgctgctt     300

tctcattgtg gccttccgcc actttcggat gataaaagtg tttctctggc tatgcggcgt     360

atccggcgcg aagcggcaac ggaaaaaggc gaacgaacag gccaggctat accgctgcga     420

tgggacgacc tgaaactgct cgacgtcctg ttgtccaggt cagaacggct ggtggacctg     480

cgcaaccggg ctttctcttt tgttgcatac aatacgctga tgcgtatgtc ggaaatctcg     540

cgtattcgtg taggagatct ggaccaaaca ggtgacactg tcacgctaca tatttcacac     600

acgaagacaa taacgaccgc cgccgggctt gataaggtgc tttcccgtcg cactactgct     660

gtgctgaatg actggctgga tgtttccggg cttcgtgaac atcctgacgc ggtgctgttc     720

ccgccgatac accgtagcaa taaggccagg attacgacaa cgccccttac tgcacctgca     780

atggagaaaa tattcagcga cgcctgggtg ttgctgaata aaagggatgc cacgccaaac     840

aaagggagat accggacgtg gaccgggcat agtgctcgtg tcggggccgc tatcgatatg     900

gctgaaaagc aggtgtctat ggtggagatt atgcaggaag gaacctggaa gaaaccggaa     960

acacttatgc ggtatctgcg ccgtggcggt gtgtctgtgg gggctaatag ccgcttgatg    1020

gattcataa                                                            1029

<210> 2
<211> 1032
<212> DNA
<213> enterobacteria phage P1

<400> 2
atgtccaatt tactgaccgt acaccaaaat ttgcctgcat taccggtcga tgcaacgagt      60

gatgaggttc gcaagaacct gatggacatg ttcagggatc gccaggcgtt ttctgagcat     120

acctggaaaa tgcttctgtc cgtttgccgg tcgtgggcgg catggtgcaa gttgaataac     180

cggaaatggt ttcccgcaga acctgaagat gttcgcgatt atcttctata tcttcaggcg 240
cgcggtctgg cagtaaaaac tatccagcaa catttgggcc agctaaacat gcttcatcgt 300
cggtccgggc tgccacgacc aagtgacagc aatgctgttt cactggttat gcggcggatc 360
cgaaaagaaa acgttgatgc cggtgaacgt gcaaaacagg ctctagcgtt cgaacgcact 420
gatttcgacc aggttcgttc actcatggaa aatagcgatc gctgccagga tatacgtaat 480
ctggcatttc tggggattgc ttataacacc ctgttacgta tagccgaaat tgccaggatc 540
agggttaaag atatctcacg tactgacggt gggagaatgt taatccatat tggcagaacg 600
aaaacgctgg ttagcaccgc aggtgtagag aaggcactta gcctgggggt aactaaactg 660
gtcgagcgat ggatttccgt ctctggtgta gctgatgatc cgaataacta cctgttttgc 720
cgggtcagaa aaaatggtgt tgccgcgcca tctgccacca gccagctatc aactcgcgcc 780
ctggaaggga tttttgaagc aactcatcga ttgatttacg gcgctaagga tgactctggt 840
cagagatacc tggcctggtc tggacacagt gcccgtgtcg gagccgcgcg agatatggcc 900
cgcgctggag tttcaatacc ggagatcatg caagctggtg gctggaccaa tgtaaatatt 960
gtcatgaact atatccgtaa cctggatagt gaaacagggg caatggtgcg cctgctggaa 1020
gatggcgatt ag 1032

<210> 3
<211> 1272
<212> DNA
<213> Saccharomyces cerevisiae

<400> 3
atgccacaat ttgatatatt atgtaaaaca ccacctaagg tgcttgttcg tcagtttgtg 60
gaaaggtttg aaagaccttc aggtgagaaa atagcattat gtgctgctga actaacctat 120
ttatgttgga tgattacaca taacggaaca gcaatcaaga gagccacatt catgagctat 180
aatactatca taagcaattc gctgagtttg gatattgtca acaagtcact gcagtttaaa 240
tacaagacgc aaaaagcaac aattctggaa gcctcattaa agaaattgat tcctgcttgg 300
gaatttacaa ttattcctta ctatggacaa aaacatcaat ctgatatcac tgatattgta 360
agtagtttgc aattacagtt cgaatcatcg gaagaagcag ataagggaaa tagccacagt 420
aaaaaaatgc ttaaagcact tctaagtgag ggtgaaagca tctgggagat cactgagaaa 480
atactaaatt cgtttgagta tacttcgaga tttacaaaaa caaaaacttt ataccaattc 540
ctcttcctag ctactttcat caattgtgga agattcagcg atattaagaa cgttgatccg 600
aaatcattta aattagtcca aaataagtat ctgggagtaa taatccagtg tttagtgaca 660
gagacaaaga caagcgttag taggcacata tacttcttta gcgcaagggg taggatcgat 720
ccacttgtat atttggatga atttttgagg aattctgaac cagtcctaaa acgagtaaat 780
aggaccggca attcttcaag caacaagcag gaataccaat tattaaaaga taacttagtc 840
agatcgtaca acaaagcttt gaagaaaaat gcgccttatt caatctttgc tataaaaaat 900

ggcccaaaat ctcacattgg aagacatttg atgacctcat ttctttcaat gaagggccta 960
acggagttga ctaatgttgt gggaaattgg agcgataagc gtgcttctgc cgtggccagg 1020
acaacgtata ctcatcagat aacagcaata cctgatcact acttcgcact agtttctcgg 1080
tactatgcat atgatccaat atcaaaggaa atgatagcat tgaaggatga gactaatcca 1140
attgaggagt ggcagcatat agaacagcta aagggtagtg ctgaaggaag catacgatac 1200
cccgcatgga atgggataat atcacaggag gtactagact acctttcatc ctacataaat 1260
agacgcatat aa 1272

<210> 4
<211> 1029
<212> DNA
<213> Artificial

<220>
<223> Dre-Rekombinase

<400> 4
atgtccgagc tgatcatctc cggctcctcc ggcggcttcc tgcgcaacat cggcaaggag 60
taccaggagg ccgccgagaa cttcatgcgc ttcatgaacg accagggcgc ctacgccccg 120
aacaccctgc gcgacctgcg cctggtcttc cactcctggg cccgctggtg ccacgcccgc 180
cagctggcct ggttcccgat ctccccggag atggcccgcg agtacttcct gcagctgcac 240
gacgccgacc tggcctccac caccatcgac aagcactacg ccatgctgaa catgctgctg 300
tcccactgcg gcctgccgcc gctgtccgac gacaagtccg tctccctggc catgcgccgc 360
atccgccgcg aggccgccac cgagaagggc gagcgcaccg gccaggccat cccgctgcgc 420
tgggacgacc tgaagctgct ggacgtcctg ctgtcccgct ccgagcgcct ggtcgacctg 480
cgcaaccgcg ccttcctgtt cgtcgcctac aacaccctga tgcgcatgtc cgagatctcc 540
cgcatccgcg tcggcgacct ggaccagacc ggcgacaccg tcaccctgca catctcccac 600
accaagacca tcaccaccgc cgccggcctg gacaaggtcc tgtcccgccg caccaccgcc 660
gtcctgaacg actggctgga cgtctccggc ctgcgcgagc acccggacgc cgtcctgttc 720
ccgccgatcc accgctccaa caaggcccgc atcaccacca ccccgctgac cgccccggcc 780
atggagaaga tcttctccga cgcctgggtc ctgctgaaca agcgcgacgc caccccgaac 840
aagggccgct accgcacctg gaccggccac tccgcccgcg tcggcgccgc catcgacatg 900
gccgagaagc aggtctccat ggtcgagatc atgcaggagg gcacctggaa gaagccggag 960
accctgatgc gctacctgcg ccgcggcggc gtctccgtcg gcgccaactc ccgcctgatg 1020
gactcctga 1029

<210> 5
<211> 1032
<212> DNA
<213> Artificial

<220>
<223> Cre-Rekombinase

<400> 5
atgtccaacc tgctgaccgt ccaccagaac ctgccggccc tgcccgtgga cgccaccagc 60

gacgaggtcc ggaagaacct gatggacatg ttccgggacc ggcaggcctt ctccgagcac 120

acctggaaga tgctgctgtc cgtgtgccgc tcgtgggccg cgtggtgcaa gctgaacaac 180

cggaagtggt tcccggcgga gccggaggac gtgcgcgact acctcctgta cctccaggcc 240

cggggcctgg cggtcaagac catccagcag cacctgggcc agctgaacat gctgcaccgc 300

cggtcgggtc tgccgcgccc cagcgacagc aacgcggtca gcctggtcat gcggcgcatc 360

cggaaggaaa acgtcgacgc cggggagcgc gccaagcagg cgctggcgtt cgagcgcacc 420

gacttcgacc aggtccgcag cctcatggag aactcggacc ggtgccagga catccggaac 480

ctggcgttcc tgggtatcgc gtacaacacc ctgctccgca tcgccgagat cgcgcgcatc 540

cgcgtgaagg acatcagccg gaccgacggt ggccgcatgc tgatccacat cgggcggacg 600

aagaccctcg tctccaccgc cggcgtcgag aaggcgctct cgctcggcgt gaccaagctc 660

gtggagcgct ggatctccgt ctccggcgtc gccgacgacc cgaacaacta cctgttctgc 720

cgcgtgcgga agaacggcgt ggccgccccc tccgcgacct cccagctctc cacgcgcgcc 780

ctggagggca tcttcgaggc cacgcaccgc ctcatctacg gggccaagga cgactccggc 840

cagcggtacc tggcgtggtc gggccactcc gcgcgcgtcg gtgccgcgcg ggacatggcc 900

cgcgcggggg tgtcgatccc ggagatcatg caggccggcg ggtggaccaa cgtcaacatc 960

gtcatgaact acatccgcaa cctggactcg gagacgggcg cgatggtgcg cctgctggaa 1020

gacggcgact ga 1032

<210> 6
<211> 1272
<212> DNA
<213> Artificial

<220>
<223> Flp-Rekombinase

<400> 6
atgccgcagt tcgacatcct ctgcaagacc ccgcccaagg tgctcgtgcg gcagttcgtc 60

gagcgcttcg agcgcccgag cggcgagaag atcgccctgt gcgccgccga gctgacctac 120

ctctgctgga tgatcaccca caacggcacg gcgatcaagc gggccacctt catgtcctac 180

aacaccatca tctccaactc gctcagcctc gacatcgtga acaagtccct ccagttcaag 240

tacaagaccc agaaggcgac catcctggaa gcgtccctga agaagctgat cccggcgtgg 300

gagttcacca tcatccccta ctacggccag aagcaccagt ccgacatcac cgacatcgtc 360

tcctcgctcc agctccagtt cgagtcgtcc gaggaggccg acaagggcaa ctcccactcc 420

aagaagatgc tgaaggcgct gctgagcgag ggcgagtcca tctgggagat cacggagaag 480

atcctgaact ccttcgagta cacctcccgg ttcaccaaga cgaagaccct ctaccagttc 540

ctgttcctgg cgaccttcat caactgcggc cggttcagcg acatcaagaa cgtcgacccg 600

aagtccttca agctggtcca gaacaagtac ctgggcgtca tcatccagtg cctggtcacc 660

gagacgaaga cctccgtgtc gcggcacatc tacttcttct ccgcgcgggg ccgcatcgac 720

ccgctggtct acctcgacga gttcctgcgc aactcggagc cggtcctcaa gcgcgtcaac 780

cggaccggca acagcagctc caacaagcag gagtaccagc tgctcaagga caacctggtc 840

cggtcgtaca acaaggccct gaagaagaac gccccgtact ccatcttcgc gatcaagaac 900

ggcccgaaga gccacatcgg tcgccacctg atgacctcgt tcctgtccat gaaggggctg 960

accgagctca ccaacgtcgt cggtaactgg agcgacaagc gcgcctccgc cgtggcgcgg 1020

accacgtaca cccaccagat cacggccatc ccggaccact acttcgccct ggtgtcgcgc 1080

tactacgcct acgacccgat ctccaaggaa atgatcgccc tgaaggacga gacgaacccg 1140

atcgaggagt ggcagcacat cgagcagctg aagggttccg ccgagggctc gatccggtac 1200

ccggcctgga acggtatcat ctcgcaggag gtgctggact acctgagcag ctacatcaac 1260

cggcggatct ga 1272

<210> 7
<211> 34
<212> DNA
<213> Artificial

<220>
<223> loxP-Erkennungsstelle

<400> 7
ataacttcgt ataatgtatg ctatacgaag ttat 34

<210> 8
<211> 32
<212> DNA
<213> Artificial

<220>
<223> Rox-Erkennungsstelle

<400> 8
taactttaaa taatgccaat tatttaaagt ta 32

<210> 9
<211> 34
<212> DNA
<213> Artificial

<220>
<223> frt-Erkennungsstelle

<400> 9
gaagttccta ttctctagaa agtataggaa cttc 34

<210> 10
<211> 342
<212> PRT
<213> enterobacteria phage D6

<400> 10

Met Ser Glu Leu Ile Ile Ser Gly Ser Ser Gly Gly Phe Leu Arg Asn
1               5                   10                  15

Ile Gly Lys Glu Tyr Gln Glu Ala Ala Glu Asn Phe Met Arg Phe Met
            20                  25                  30

Asn Asp Gln Gly Ala Tyr Ala Pro Asn Thr Leu Arg Asp Leu Arg Leu
        35                  40                  45

Val Phe His Ser Trp Ala Arg Trp Cys His Ala Arg Gln Leu Ala Trp
    50                  55                  60

Phe Pro Ile Ser Pro Glu Met Ala Arg Glu Tyr Phe Leu Gln Leu His
65                  70                  75                  80

Asp Ala Asp Leu Ala Ser Thr Thr Ile Asp Lys His Tyr Ala Met Leu
                85                  90                  95

Asn Met Leu Leu Ser His Cys Gly Leu Pro Pro Leu Ser Asp Asp Lys
            100                 105                 110

Ser Val Ser Leu Ala Met Arg Arg Ile Arg Arg Glu Ala Ala Thr Glu
        115                 120                 125

Lys Gly Glu Arg Thr Gly Gln Ala Ile Pro Leu Arg Trp Asp Asp Leu
    130                 135                 140

Lys Leu Leu Asp Val Leu Leu Ser Arg Ser Glu Arg Leu Val Asp Leu
145                 150                 155                 160

Arg Asn Arg Ala Phe Leu Phe Val Ala Tyr Asn Thr Leu Met Arg Met
                165                 170                 175

Ser Glu Ile Ser Arg Ile Arg Val Gly Asp Leu Asp Gln Thr Gly Asp
            180                 185                 190

Thr Val Thr Leu His Ile Ser His Thr Lys Thr Ile Thr Thr Ala Ala
        195                 200                 205

Gly Leu Asp Lys Val Leu Ser Arg Arg Thr Thr Ala Val Leu Asn Asp
    210                 215                 220

Trp Leu Asp Val Ser Gly Leu Arg Glu His Pro Asp Ala Val Leu Phe
225                 230                 235                 240

Pro Pro Ile His Arg Ser Asn Lys Ala Arg Ile Thr Thr Thr Pro Leu
                245                 250                 255

Thr Ala Pro Ala Met Glu Lys Ile Phe Ser Asp Ala Trp Val Leu Leu
            260                 265                 270

Asn Lys Arg Asp Ala Thr Pro Asn Lys Gly Arg Tyr Arg Thr Trp Thr
        275                 280                 285

Gly His Ser Ala Arg Val Gly Ala Ala Ile Asp Met Ala Glu Lys Gln
    290                 295                 300

Val Ser Met Val Glu Ile Met Gln Glu Gly Thr Trp Lys Lys Pro Glu
305                 310                 315                 320

Thr Leu Met Arg Tyr Leu Arg Arg Gly Gly Val Ser Val Gly Ala Asn
                325                 330                 335

Ser Arg Leu Met Asp Ser
            340

<210>  11
<211>  343
<212>  PRT
<213>  retrofitting vector pRetroES

<400>  11

Met Ser Asn Leu Leu Thr Val His Gln Asn Leu Pro Ala Leu Pro Val
1               5                   10                  15

Asp Ala Thr Ser Asp Glu Val Arg Lys Asn Leu Met Asp Met Phe Arg
            20                  25                  30

Asp Arg Gln Ala Phe Ser Glu His Thr Trp Lys Met Leu Leu Ser Val
        35                  40                  45

Cys Arg Ser Trp Ala Ala Trp Cys Lys Leu Asn Asn Arg Lys Trp Phe
    50                  55                  60

Pro Ala Glu Pro Glu Asp Val Arg Asp Tyr Leu Leu Tyr Leu Gln Ala
65                  70                  75                  80

Arg Gly Leu Ala Val Lys Thr Ile Gln Gln His Leu Gly Gln Leu Asn
                85                  90                  95

Met Leu His Arg Arg Ser Gly Leu Pro Arg Pro Ser Asp Ser Asn Ala
            100                 105                 110

Val Ser Leu Val Met Arg Arg Ile Arg Lys Glu Asn Val Asp Ala Gly
        115                 120                 125

Glu Arg Ala Lys Gln Ala Leu Ala Phe Glu Arg Thr Asp Phe Asp Gln
    130                 135                 140

Val Arg Ser Leu Met Glu Asn Ser Asp Arg Cys Gln Asp Ile Arg Asn
145                 150                 155                 160

Leu Ala Phe Leu Gly Ile Ala Tyr Asn Thr Leu Leu Arg Ile Ala Glu

                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Lys Asn Gly Val Ala Ala Pro Ser Ala Thr Ser Gln Leu
                245                 250                 255

Ser Thr Arg Ala Leu Glu Gly Ile Phe Glu Ala Thr His Arg Leu Ile
            260                 265                 270

Tyr Gly Ala Lys Asp Asp Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Ser Ile Pro Glu Ile Met Gln Ala Gly Gly Trp Thr Asn Val Asn Ile
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210> 12
<211> 423
<212> PRT
<213> Saccharomyces cerevisiae

<400> 12

Met Pro Gln Phe Asp Ile Leu Cys Lys Thr Pro Pro Lys Val Leu Val
1               5                   10                  15

Arg Gln Phe Val Glu Arg Phe Glu Arg Pro Ser Gly Glu Lys Ile Ala
            20                  25                  30

Leu Cys Ala Ala Glu Leu Thr Tyr Leu Cys Trp Met Ile Thr His Asn
        35                  40                  45

Gly Thr Ala Ile Lys Arg Ala Thr Phe Met Ser Tyr Asn Thr Ile Ile
    50                  55                  60

Ser Asn Ser Leu Ser Leu Asp Ile Val Asn Lys Ser Leu Gln Phe Lys
65                  70                  75                  80

Tyr Lys Thr Gln Lys Ala Thr Ile Leu Glu Ala Ser Leu Lys Lys Leu
                85                  90                  95

Ile Pro Ala Trp Glu Phe Thr Ile Ile Pro Tyr Tyr Gly Gln Lys His
            100                 105                 110

Gln Ser Asp Ile Thr Asp Ile Val Ser Ser Leu Gln Leu Gln Phe Glu
        115                 120                 125

Ser Ser Glu Glu Ala Asp Lys Gly Asn Ser His Ser Lys Lys Met Leu
    130                 135                 140

Lys Ala Leu Leu Ser Glu Gly Glu Ser Ile Trp Glu Ile Thr Glu Lys
145                 150                 155                 160

Ile Leu Asn Ser Phe Glu Tyr Thr Ser Arg Phe Thr Lys Thr Lys Thr
                165                 170                 175

Leu Tyr Gln Phe Leu Phe Leu Ala Thr Phe Ile Asn Cys Gly Arg Phe
            180                 185                 190

Ser Asp Ile Lys Asn Val Asp Pro Lys Ser Phe Lys Leu Val Gln Asn
        195                 200                 205

Lys Tyr Leu Gly Val Ile Ile Gln Cys Leu Val Thr Glu Thr Lys Thr
    210                 215                 220

Ser Val Ser Arg His Ile Tyr Phe Phe Ser Ala Arg Gly Arg Ile Asp
225                 230                 235                 240

Pro Leu Val Tyr Leu Asp Glu Phe Leu Arg Asn Ser Glu Pro Val Leu
                245                 250                 255

Lys Arg Val Asn Arg Thr Gly Asn Ser Ser Ser Asn Lys Gln Glu Tyr
            260                 265                 270

Gln Leu Leu Lys Asp Asn Leu Val Arg Ser Tyr Asn Lys Ala Leu Lys
        275                 280                 285

Lys Asn Ala Pro Tyr Ser Ile Phe Ala Ile Lys Asn Gly Pro Lys Ser
    290                 295                 300

His Ile Gly Arg His Leu Met Thr Ser Phe Leu Ser Met Lys Gly Leu
305                 310                 315                 320

Thr Glu Leu Thr Asn Val Val Gly Asn Trp Ser Asp Lys Arg Ala Ser
                325                 330                 335

Ala Val Ala Arg Thr Thr Tyr Thr His Gln Ile Thr Ala Ile Pro Asp
            340                 345                 350

His Tyr Phe Ala Leu Val Ser Arg Tyr Tyr Ala Tyr Asp Pro Ile Ser
        355                 360                 365

Lys Glu Met Ile Ala Leu Lys Asp Glu Thr Asn Pro Ile Glu Glu Trp
    370                 375                 380

Gln His Ile Glu Gln Leu Lys Gly Ser Ala Glu Gly Ser Ile Arg Tyr
385                 390                 395                 400

Pro Ala Trp Asn Gly Ile Ile Ser Gln Glu Val Leu Asp Tyr Leu Ser
                405                 410                 415

Ser Tyr Ile Asn Arg Arg Ile
            420